# EUROPEAN PATENT SPECIFICATION

(11) **EP 1 477 557 B1**
(45) Date of publication and mention of the grant of the patent: **30.04.2008**
(21) Application number: 04016329.7
(22) Date of filing: 29.03.2000
(51) Int. Cl.: C12N 9/10, C12N 15/54, C12N 15/29, C12N 15/82, C12N 15/63, C12N 5/04, A01H 5/00

(54) **Transgenic plant resistant to mycotoxins and methods of production and use**
Mykotoxin-resistente transgene Pflanze und Verfahren zur Herstellung und Verwendung derselben
Plante transgénique résistant aux mycotoxines, procédés de production et d'utilisation

(30) Priority: 31.03.1999 US 282995; 11.02.2000 US 502852
(43) Date of publication of application: 17.11.2004
(62) Divisional of application: 00926773.3
(73) Proprietor: Syngenta Participations AG, 4058 Basel (CH)
(72) Inventor: Hohn, Thomas M., Research Triangle Park N. Carolina 27709 (US); Peters, Cheryl, Research Triangle Park N. Carolina 27709 (US); Salmeron, John Manuel, Research Triangle Park N. Carolina 27709 (US); Reed, Janet N., Research Triangle Park N. Carolina 27709 (US); Dawson, John Luther, Research Triangle Park N. Carolina 27709 (US)
(74) Representative: Kock, Michael Andreas

(56) References cited:
- WO-A-99/09173
- KIMURA MAKOTO ET AL: "The mystery of the trichothecene 3-O-acetyltransferase gene. Analysis of the region around Tri101 and characterization of its homologue form Fusarium sporotrichioides." FEBS LETTERS, vol. 435, no. 2-3, 1998, pages 163-168, XP000946098 ISSN: 0014-5793
- KIMURA MAKOTO ET AL: "Features of Tri101, the trichothecene 3-0-acetyltransferase gene, related to the self-defense mechanism in Fusarium graminearum." BIOSCIENCE BIOTECHNOLOGY AND BIOCHEMISTRY, vol. 62, no. 5, May 1998 (1998-05), pages 1033-1036, XP000946099 ISSN: 0916-8451
- KIMURA M ET AL: "Trichothecene 3-O-acetyltransferase protects both the producing organism and transformed yeast from related mycotoxins. Cloning and characterization of Tri101" JOURNAL OF BIOLOGICAL CHEMISTRY,US,AMERICAN SOCIETY OF BIOLOGICAL CHEMISTS, BALTIMORE, MD, vol. 273, no. 3, 16 January 1998 (1998-01-16), pages 1654-1661, XP002125091 ISSN: 0021-9258
- DATABASE EMBL NUCLEOTIDE AND PROTEIN SEQUENCES, 28 May 1996 (1996-05-28), XP002147697 HINXTON, GB
- MCCORMICK SUSAN P ET AL: "Disruption of TRI101, the gene encoding trichothecene 3-O-acetyltransferase, from Fusarium sporotrichioides." APPLIED AND ENVIRONMENTAL MICROBIOLOGY, vol. 65, no. 12, December 1999 (1999-12), pages 5252-5256, XP000938222 ISSN: 0099-2240
- DATABASE WPI Section Ch, Week 200024 Derwent Publications Ltd., London, GB; Class C06, AN 2000-274037 XP002147698 & JP 2000 032985 A (RIKAGAKU KENKYUSHO) 2 February 2000 (2000-02-02)

## Description

The present invention relates to transgenic hosts particularly transgenic plants, plant tissues, seeds and cells that are trichothecene resistant and methods of making and using the same. The present invention further relates to methods of preventing and/or reducing fungal growth on a plant, plant tissue, seed or plant cell. The present invention further relates to preventing and/or reducing mycotoxin contamination of a plant, plant tissue or seed. The present invention further relates to using trichothecenes as selective agents in transformation protocols.

Numerous fungi are serious pests of economically important agricultural crops. Further, crop contamination by fungal toxins is a major problem for agriculture throughout the world. Mycotoxins are toxic fungal metabolites, often found in agricultural products that are characterized by their ability to cause health problems for vertebrates. Trichothecenes are sesquiterpene epoxide mycotoxins produced by species of *Fusarium, Trichothecium,* and *Myrothecium* that act as potent inhibitors of eukaryotic protein synthesis. *Fusarium* species that produce such trichothecenes include *F. acuminatum, F. crookwellense, F. culmorum, F. equiseti, F. graminearum (Gibberella zeae), F. lateritium, F. poae, F. sambucinum (G. pulicaris),* and *F. sporotrichioides* (Marasas, W.F.O., Nelson, P.E., and Toussoun, T.A. 1984).
As previously described (A. E.Desjardins and T. M Hohn, Mycotoxins in plant pathogenesis.Mol.Plant-Microbe Interact. 10 (2):147-152, 1997), both acute and chronic mycotoxicoses in farm animals and in humans have been associated with consumption of wheat, rye, barley, oats, rice and maize contaminated with *Fusarium* species that produce trichothecene mycotoxins. Experiments with chemically pure trichothecenes at low dosage levels have reproduced many of the features observed in moldy-grain toxicoses in animals, including anemia and immunosuppression, hemorrage, emesis and feed refusal. Historical and epidemiological data from human populations indicate an association between certain disease epidemics and consumption of grain infected with *Fusarium* species that produce trichothecenes. In particular, outbreaks of a fatal disease known as alimentary toxic aleukia, which has occurred in Russia since the nineteenth century, have been associated with consumption of over-wintered grains contaminated with *Fusarium* species that produce the trichothecene T-2 toxin. In Japan, outbreaks of a similar disease called akakabi-byo or red mold disease have been associated with grain infected with *Fusarium* species that produce the trichothecene, deoxynivalenol (hereinafter "DON"). Trichothecenes were detected in the toxic grain samples responsible for recent human disease outbreaks in India and Japan. There exists, therefore, a need for agricultural methods for preventing and, crops having reduced levels of, mycotoxin contamination.
Further, trichothecene-producing *Fusarium* species are destructive pathogens and attack a wide range of plant species. The acute phytotoxicity of trichothecenes and their occurrence in plant tissues also suggest that these mycotoxins play a role in the pathogenesis of *Fusarium* on plants. This implies that mycotoxins play a role in disease and, therefore, reducing their toxicity to the plant may also prevent or reduce disease in the plant. Further, reduction in disease levels may have the additional benefit of reducing mycotoxin contamination on the plant and particularly in grain where the plant is a cereal plant.
Various methods of controlling diseases in plants, such as corn ear rot, stock rot or wheat head blight, have been used with varying degrees of success. One method of controlling plant disease has been to apply an antimicrobial chemical to crops. This method has numerous, art-recognized problems. Alternatively, a more recent method involves the use of biological control organisms ("biocontrol") which are natural competitors or inhibitors of the pest organism. However, it is difficult to apply biocontrol to large areas, and even more difficult to cause those living organisms to remain in the treated area for an extended period of time. More recently, techniques in recombinant DNA have provided the opportunity to insert into plant cells cloned genes, which express antimicrobial compounds. However, this technology has given rise to concerns about eventual microbial resistance to well-known, naturally occurring antimicrobials. Thus, a continuing need exists to identify naturally occurring antimicrobial agents, such as proteins, which can be formed by plant cells directly by translation of a single gene.

A trichothecene 3-O-acetyltransferase that catalyzes the acetylation of a number of different *Fusarium* trichothecenes including DON at the C3 hyroxyl group has been identified in *Fusarium sporotrichioides.* (S. P. McCormick, N. J. Alexander, S. C. Trapp, and T. M. Hohn. Disruption of TRI101, the gene encoding trichothecene 3-O-acetyltransferase, from Fusarium sporotrichioides. Applied.Environ.Microbiol. 65 (12):5252-5256, 1999.) Acetylation of trichothecenes at the C3-OH significantly reduces their toxicity in vertebrates and plants and results in the reaction product 3-acetyldeoxyvalenol (hereinafter "3ADON") See, Kimura et al. below.

The sequence of structural genes encoding trichothecene 3-O-acetyl transferases from *Fusarium graminearum, Fusarium sporotrichioides* as well as sequences of other orthologs has been published. See, e.g. Kimura et al., Biosci. Biotechnol. Biochem., 62 (5) 1033-1036 (1998), and Kimura et al., FEBS Letters, 435 , 163-168 (1998). Further, it has been speculated that the gene from *Fusarium sporotrichioides* encoding a trichothecene 3-O-acetyl transferase may be useful in developing plant varieties with increased resistance to *Fusarium.* See., e.g. Hohn, T.M. et al. Molecular Genetics of Host-Specific Toxins in Plant Disease, 17-24 (1998), and Kimura et al. J.Biological Chemistry, 273(3) 1654-1661 (1998).

Prior to the present invention, however, many uncertainties rendered it far from obvious whether expressing trichothecene 3-O-acetyl transferases in a plant would actually lead to trichothecene resistant plants. For example, the reaction catalyzed by the *Fusarium sporotrichoides* trichothecene 3-O-acetyl transferase is reversible and might, therefore have failed to protect plant cells from trichothecenes such as DON. It was also uncertain whether there might be esterases in plant cells that would compete with the 3-O-acetyl transferase activities to generate toxic DON from 3ADON. It was also uncertain how the metabolism of the reaction product 3ADON might affect the plant, e.g. whether introduction of the trichothecene 3-O- acetyltransferase would alter plant growth and development in ways that would negate any positive contribution of the acetyltransferase by for example, interfering with the plant's natural disease resistance mechanisms. It was also uncertain whether 3ADON could be metabolized by the plant to form a novel secondary metabolite with toxic effects. It was also uncertain, even if DON produced by an invading fungus was efficiently converted to 3ADON, whether this conversion would impart enhanced pathogen resistance upon the plant. The above are but a few of the uncertainties in the art before the time of the present invention.

It is an object of the invention to provide a plant cell or cells comprising a heterologous polynucleotide encoding a gene product that is expressed in the plant cell wherein the gene product comprises trichothecene resistance activity.
Another object of the invention is to provide a plant comprising the above described plant cell wherein the plant is resistant to a trichothecene.
Another object of the invention is to provide a plant of the invention that is resistant to a trichothecene where the trichothecene comprises a C-3 hydroxyl group.

Another object of the invention is to provide a plant of the invention that is resistant to a fungus that produces a trichothecene, preferably a trichothecene comprising a C-3 hydroxyl group.

Another object of the invention is to provide a plant of the invention that is resistant to *Fusarium, Trichothecium or Myrothecium.*

Another object of the invention is to provide a plant of the invention that is resistant to *Fusarium,* in particular but not limited to *Fusarium graminearum, Fusarium culmorum, Fusarium sporotrichioides, Fusarium poae, Fusarium sambucinum, Fusarium equiseti, Fusarium acuminatum, Fusarium lateritium,* and *Fusarium pseudograminearum.*

Another object of the invention is to provide a plant of the invention, wherein the plant is resistant to *Fusarium graminearum.*

Another object of the invention is to provide a plant of the invention comprising a heterologous polynucleotide encoding a gene product that is expressed in the plant cell wherein the gene product comprises trichothecene resistance activity, wherein the heterologous polynucleotide is a microbial polynucleotide, preferably a a yeast or fungal polynucleotide.

Another object of the invention is to provide a plant of the invention, wherein the fungal polynucleotide is a *Fusarium* polynucleotide, preferably is a *Fusarium graminearum* or *Fusarium sporotrichioides* polynucleotide.

Another object of the invention is to provide a plant of the invention wherein the heterologous polynucleotide comprises a sequence substantially similar to the nucleic acid sequence of SEQ ID NOs:1, 5 or 7.

Another object of the invention is to provide a plant of the invention wherein the heterologous polynucleotide comprises the nucleic acid sequence of SEQ ID NO:1 5 or 7 or homologs thereof.

Another object of the invention is to provide a plant of the invention comprising a heterologous polynucleotide, which comprises a consecutive at least 80 base pair portion identical in sequence to a consecutive 80 base pair portion set forth in SEQ ID NO:1, 5 or 7, preferably a consecutive at least 50 base pair portion identical in sequence to a consecutive 50 base pair portion set forth in SEQ ID NO:1, 5 or 7, and more preferably a consecutive at least 21 base pair portion identical in sequence to a consecutive 21 base pair portion set forth in SEQ ID NO:1, 5 or 7, and most preferably a consecutive 18 base pair portion identical in sequence to a consecutive 18 base pair portion set forth in SEQ ID NO:1, 5 or 7.

Another object of the invention is to provide a plant of the invention resistant to a trichothecene selected from the group consisting T-2 toxin, HT-2 toxin, isotrichodermol, 4,15-diacetoxyscirpenol (hereinafter "DAS"), 3-deacetylcalonectrin, 3,15-dideacetylcalonectrin scirpentriol, neosolaniol; type B: 15-acetyldeoxynivalenol, nivalenol, 4-acetylnivalenol (fusarenone-X), 4,15-diacetylnivalenol, 4,7,15-acetylnivalenol, and DON.
Another object of the invention is to provide a plant of the invention resistant to DAS or DON.

Another object of the invention is to provide a plant wherein the gene product is encoded by a polynucleotide according to the invention.

Another object of the invention is to provide a plant of the invention wherein the gene product is a 3-O-acetyltransferase.

Another object of the invention is to provide a plant of the invention wherein the gene product is a polypeptide comprising a sequence substantially similar to SEQ ID NO:2, 6 or 8.

Another object of the invention is to provide a seed of any of the plants of the invention.

Another object of the invention is to provide anyone of the above-described plants wherein the plant is a wheat, maize, barley or rice plant.

Another object of the invention is to provide a method for producing a trichothecene resistant plant comprising the steps of:
(a) transforming a plant cell with a heterologous gene encoding a gene product , wherein the gene product increases resistance to a trichothecene; and
(b)expressing the gene product at a biologically significant level.
(c) regenerating the plant cell into a plant; and
(d)selecting a plant having increased resistance to a trichothecene.

Another object of the invention is to provide a method as described above further comprising the step of selecting a plant on which there is reduced growth of a fungus where the fungus produces a trichothecene.

Another object of the invention is to provide a method as described above wherein the fungus is of the genus *Fusarium.*

Another object of the invention is to provide a trichothecene resistant plant obtained according to the above-described methods.

Another object of the invention is to provide a seed produced by selfing or outcrossing a plant of the invention as described above, wherein a plant grown from the seed has an increased resistance to trichothecene.

Another object of the invention is to provide a method of preventing mycotoxin contamination of a plant, including of a plant's seed, comprising growing a plant of the invention as described above, preferably in an area with moderate to severe fungal infestation, wherein the plant is preferably a crop plant.

Another object of the invention is to provide a method of preventing fungal growth on a plant, preferably growth of a fungus of the genus *Fusarium* comprising growing a plant of the invention that produces a trichothecene, preferbably a trichothece comprising a C-3 hydroxyl group as described above, preferably in an area with moderate to severe fungal infestation, wherein the plant is preferably a crop plant.

Another object of the invention is to provide a method of producing a fungal resistant plant comprising
(a) transforming a plant cell with a heterologous gene encoding a gene product, wherein the gene product increases resistance to a trichothecene;
(b) expressing the gene product at a biologically significant level;
(c) regenerating the plant cell into a plant; and
(d) selecting a plant with increased resistance to a trichothecene; and
(e) optionally, selfing or outcrossig the plant obtained in step (d)

Another object of the invention is to provide the method, wherein the fungus is of the genus *Fusarium.*

Another object of the invention is to provide a method of producing seed wherein the plant grown from the seed is fungal resistant, comprising selfing or outcrossing the plant of the invention.

Another object of the invention is to provide the above method, wherein the seed is resistant to fungi to the genus *Fusarium.*

Another object of the invention is to provide a method of selecting transformed host cells, the method comprising:
transforming a host cell with a nucleic acid construct encoding a trichothecene 3-*O-*acetyltransferase, and
growing the transformed host cell in the presence of a trichothecene selective agent.

Another object of the invention is to provide a method of selecting transformed host cells wherein the host cells are plant cells, or microbial cells, particularly where the microbial cells are fungal cells.

Another object of the invention is to provide a method of selecting transformed host cells as described above where the host cell is further transformed with a second polynucleotide of interest.

Another object of the invention is to provide a method of selecting transformed host cells wherein in the trichothecene is selected from the group the group consisting T-2 toxin, HT-2 toxin, isotrichodermol, DAS, 3-deacetylcalonectrin, 3,15-dideacetylcalonectrin, scirpentriol, neosolaniol; type B: 15-acetyldeoxynivalenol, nivalenol, 4-acetylnivalenol (fusarenone-X), 4,15-diacetylnivalenol, 4,7,15-acetylnivalenol, and DON.

Another object of the invention is to provide a trichothecene resistant plant obtainable by the method of the invention.
Another object of the invention is to provide a fungal resistant plant obtainable by the method of the invention.
Another object of the invention is to provide a plant seed obtainable by the method of the invention.

For clarity, certain terms used in the specification are defined and presented as follows:
"Expression" refers to the transcription and/or translation of an endogenous gene or a transgene in plants. In the case of antisense constructs, for example, expression may refer to the transcription of the antisense DNA only.
"Operably linked/associated" when referring to a regulatory DNA sequence being"operably linked to" or "associated with" a DNA sequence that codes for an RNA or a protein refers to the two sequences being situated such that the regulatory DNA sequence affects expression of the coding DNA sequence.
The term "heterologous polynucleotide" or "heterologous DNA" as used herein each refers to a nucleic acid molecule not naturally associated with a host cell into which it is introduced, including genetic constructs, non-naturally occurring multiple copies of a naturally occurring nucleic acid molecule; and an otherwise homologous nucleic acid molecule operatively linked to a non-native nucleic acid molecule. Thus, a heterologous gene in a host cell includes a gene that is endogenous to the particular host cell but has been modified through, for example, the use of DNA shuffling. Thus, the terms encompasses a DNA segment that is foreign or heterologous to the cell, or homologous to the cell but in a position within the host cell nucleic acid in which the element is not ordinarily found.
The terms "nucleic acid" or "polynucleotide" refers to deoxyribonucleotides or ribonucleotides and polymers thereof in either single- or double-stranded form. Unless specifically limited, the term encompasses nucleic acids containing known analogues of natural nucleotides which have similar binding properties as the reference nucleic acid and are metabolized in a manner similar to naturally occurring nucleotides. Unless otherwise indicated, a particular nucleic acid sequence also implicitly encompasses conservatively modified variants thereof (e.g. degenerate codon substitutions) and complementary sequences and as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19: 5081 (1991); Ohtsuka et al., J. Biol. Chem. 260: 2605-2608 (1985); Rossolini et al., Mol. Cell. Probes 8: 91-98 (1994)). The terms "nucleic acid" or "nucleic acid sequence" or "polynucleotide" may also be used interchangeably with gene, cDNA, and mRNA encoded by a gene.

In its broadest sense, the term "substantially similar", when used herein with respect to a nucleic acid molecule, means a nucleic acid molecule corresponding to a reference nucleotide sequence, wherein the corresponding nucleic acid molecule encodes a polypeptide having substantially the same structure and function as the polypeptide encoded by the reference nucleotide sequence, e.g. where only changes in amino acids not affecting the polypeptide function occur. Desirably the substantially similar nucleic acid molecule encodes the polypeptide encoded by the reference nucleotide sequence. The term "substantially similar" is specifically intended to include nucleic acid molecules wherein the sequence has been modified to optimize expression in particular cells, e.g. in plant cells. The percentage of identity between the substantially similar nucleic acid molecule and the reference nucleotide sequence desirably is at least 45%, more desirably at least 65%, more desirably at least 75%, preferably at least 85%, more preferably at least 90%, still more preferably at least 95%; yet still more preferably at least 99%. Preferably, the percentage of identity exists over a region of the sequences that is at least about 50 residues in length, more preferably over a region of at least about 100 residues, and most preferably the sequences are substantially similar over at least about 150 residues. In a most preferred embodiment, the sequences are substantially similar over the entire length of the coding regions. Sequence comparisons may be carried out using a Smith-Waterman sequence alignment algorithm and as described in more detail below (see e.g. Waterman, M.S. Introduction to Computational Biology: Maps, sequences and genomes. Chapman & Hall. London: 1995. ISBN 0-412-99391-0, or at http://www-hto.usc.edu/software/segain/index.html). The local S program, version 1.16, is used with following parameters: match: 1, mismatch penalty: 0.33, open-gap penalty: 2, extended-gap penalty: 2.
Another indication that a nucleic acid sequence is a substantially similar nucleic acid of the invention is that it hybridizes to a nucleic acid molecule of the invention under stringent conditions. The phrase "hybridizing specifically to" refers to the binding, duplexing, or hybridizing of a molecule only to a particular nucleotide sequence under stringent conditions when that sequence is present in a complex mixture (e.g., total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target nucleic acid sequence.
"Stringent hybridization conditions" and "stringent hybridization wash conditions" in the context of nucleic acid hybridization experiments such as Southern and Northern hybridizations are sequence dependent, and are different under different environmental parameters. Longer sequences hybridize specifically at higher temperatures. An extensive guide to the hybridization of nucleic acids is found in Tijssen (1993) Laboratory Techniques in Biochemistry and Molecular Biology-Hybridization with Nucleic Acid Probes part I chapter 2 "Overview of principles of hybridization and the strategy of nucleic acid probe assays" Elsevier, New York. Generally, highly stringent hybridization and wash conditions are selected to be about 5°C lower than the thermal melting point (Tₘ) for the specific sequence at a defined ionic strength and pH. Typically, under "stringent conditions" a probe will hybridize to its target subsequence, but to no other sequences.
The Tₘ is the temperature (under defined ionic strength and pH) at which 50% of the target sequence hybridizes to a perfectly matched probe. Very stringent conditions are selected to be equal to the Tₘ for a particular probe. An example of stringent hybridization conditions for hybridization of complementary nucleic acids which have more than 100 complementary residues on a filter in a Southern or northern blot is 50% formamide with 1 mg of heparin at 42°C, with the hybridization being carried out overnight. An example of highly stringent wash conditions is 0.15M NaCl at 72°C for about 15 minutes. An example of stringent wash conditions is a 0.2x SSC wash at 65°C for 15 minutes (see, Sambrook, *infra,* for a description of SSC buffer). Often, a high stringency wash is preceded by a low stringency wash to remove background probe signal. An example medium stringency wash for a duplex of, e.g., more than 100 nucleotides, is 1x SSC at 45°C for 15 minutes. An example low stringency wash for a duplex of, e.g., more than 100 nucleotides, is 4-6x SSC at 40°C for 15 minutes. For short probes (*e.g.*, about 10 to 50 nucleotides), stringent conditions typically involve salt concentrations of less than about 1.0M Na ion, typically about 0.01 to 1.0 M Na ion concentration (or other salts) at pH 7.0 to 8.3, and the temperature is typically at least about 30°C. Stringent conditions can also be achieved with the addition of destabilizing agents such as formamide. In general, a signal to noise ratio of 2x (or higher) than that observed for an unrelated probe in the particular hybridization assay indicates detection of a specific hybridization. Nucleic acids that do not hybridize to each other under stringent conditions are still substantially similar if the proteins that they encode are substantially similar. This occurs, e.g., when a copy of a nucleic acid is created using the maximum codon degeneracy permitted by the genetic code.
The following are examples of sets of hybridization/wash conditions that may be used to identify homologous nucleotide sequences that are substantially similar to reference nucleotide sequences of the present invention: a test sequence that hybridizes to the reference nucleotide sequence in 7% sodium dodecyl sulfate (SDS), 0.5 M NaPO₄, 1 mM EDTA at 50°C with washing in 2X SSC, 0.1% SDS at 50°C, more desirably in 7% sodium dodecyl sulfate (SDS), 0.5 M NaPO₄, 1 mM EDTA at 50°C with washing in 1X SSC, 0.1% SDS at 50°C, more desirably still in 7% sodium dodecyl sulfate (SDS), 0.5 M NaPO₄, 1 mM EDTA at 50°C with washing in 0.5X SSC, 0.1% SDS at 50°C, preferably in 7% sodium dodecyl sulfate (SDS), 0.5 M NaPO₄, 1 mM EDTA at 50°C with washing in 0.1X SSC, 0.1% SDS at 50°C, more preferably in 7% sodium dodecyl sulfate (SDS), 0.5 M NaPO₄, 1 mM EDTA at 50°C with washing in 0.1X SSC, 0.1% SDS at 65°C. The polynucleotide of the invention that hybridize under the above conditions preferably comprises at least 80 base pairs, more preferably at least 50 base pairs and particularly at least 21, and more particularly 18 base pairs. Preferred homologs of use in the invention include nucleic acid molecules that encode an amino acid sequence that is at least 45% identical to SEQ ID NO:2, 6 or 8 as measured, using the parameters described below, wherein the amino acid sequence encoded by the homolog has trichothecene resistance activity, e.g. 3-O-acetly transferase activity.
The term "substantially similar", when used herein with respect to a protein, means a protein corresponding to a reference protein, wherein the protein has substantially the same structure and function as the reference protein, e.g. where only changes in amino acids sequence not affecting the polypeptide function occur. When used for a protein or an amino acid sequence the percentage of identity between the substantially similar and the reference protein or amino acid sequence desirably is at least 45% identity, more desirably at least 65%, more desirably at least 75%, preferably at least 85%, more preferably at least 90%, still more preferably at least 95%, yet still more preferably at least 99%, using default BLAST analysis parameters and as described in more detail below.
Preferred homologs of the polypeptide of use in the invention comprise those having amino acid sequences that are at least 45% identical to SEQ ID NO:2, 6 or 8, wherein the amino acid sequence encoded by the homolog has trichothecene resistance activity, e.g. 3-*O*-acetyl transferase activity.
Optimal alignment of nucleic acid or protein sequences for comparison can be conducted as described above and, e.g., by the local homology algorithm of Smith & Waterman, Adv. Appl. Math. 2: 482 (1981), by the homology alignment algorithm of Needleman & Wunsch, J. Mol. Biol. 48: 443 (1970), by the search for similarity method of Pearson & Lipman, Proc. Nat'l. Acad. Sci. USA 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by visual inspection (see *generally,* Ausubel *et al., infra).*
One example of an algorithm that is suitable for determining percent sequence identity and sequence similarity is the BLAST algorithm, which is described in Altschul et al., J. Mol. Biol. 215: 403-410 (1990). Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al.,* 1990). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when the cumulative alignment score falls off by the quantity X from its maximum achieved value, the cumulative score goes to zero or below due to the accumulation of one or more negative-scoring residue alignments, or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (see Henikoff & Henikoff, Proc. Natl. Acad. Sci. USA 89: 10915 (1989)).
In addition to calculating percent sequence identity, the BLAST algorithm also performs a statistical analysis of the similarity between two sequences (see, *e.g.,* Karlin & Altschul, Proc. Nat'l. Acad. Sci. USA 90: 5873-5787 (1993)). One measure of similarity provided by the BLAST algorithm is the smallest sum probability (P(N)), which provides an indication of the probability by which a match between two nucleotide or amino acid sequences would occur by chance. For example; a test nucleic acid sequence is considered similar to a reference sequence if the smallest sum probability in a comparison of the test nucleic acid sequence to the reference nucleic acid sequence is less than about 0.1, more preferably less than about 0.01, and most preferably less than about 0.001.
A further indication that two nucleic acid sequences or proteins are substantially similar is that the protein encoded by the first nucleic acid is immunologically cross reactive with, or specifically binds to, the protein encoded by the second nucleic acid. Thus, a protein is typically substantially similar to a second protein, for example, where the two proteins differ only by conservative substitutions.
The phrase "specifically (or selectively) binds to an antibody," or "specifically (or selectively) immunoreactive with," when referring to a protein or peptide, refers to a binding reaction which is determinative of the presence of the protein in the presence of a heterogeneous population of proteins and other biologics. Thus, under designated immunoassay conditions, the specified antibodies bind to a particular protein and do not bind in a significant amount to other proteins present in the sample. Specific binding to an antibody under such conditions may require an antibody that is selected for its specificity for a particular protein. For example, antibodies raised to the protein with the amino acid sequence encoded by any of the nucleic acid sequences of the invention can be selected to obtain antibodies specifically immunoreactive with that protein and not with other proteins except for polymorphic variants. A variety of immunoassay formats may be used to select antibodies specifically immunoreactive with a particular protein. For example, solid-phase ELISA immunoassays, Western blots, or immunohistochemistry are routinely used to select monoclonal antibodies specifically immunoreactive with a protein. See Harlow and Lane (1988) Antibodies, A Laboratory Manual, Cold Spring Harbor Publications, New York "Harlow and Lane"), for a description of immunoassay formats and conditions that can be used to determine specific immunoreactivity. Typically a specific or selective reaction will be at least twice background signal or noise and more typically more than 10 to 100 times background.
"Conservatively modified variations" of a particular nucleic acid sequence refers to those nucleic acid sequences that encode identical or essentially identical amino acid sequences, or where the nucleic acid sequence does not encode an amino acid sequence, to essentially identical sequences. Because of the degeneracy of the genetic code, a large number of functionally identical nucleic acids encode any given polypeptide. For instance the codons CGT, CGC, CGA, CGG, AGA, and AGG all encode the amino acid arginine. Thus, at every position where an arginine is specified by a codon, the codon can be altered to any of the corresponding codons described without altering the encoded protein. Such nucleic acid variations are "silent variations" which are one species of "conservatively modified variations." Every nucleic acid sequence described herein which encodes a protein also describes every possible silent variation, except where otherwise noted. One of skill will recognize that each codon in a nucleic acid (except ATG, which is ordinarily the only codon for methionine) can be modified to yield a functionally identical molecule by standard techniques. Accordingly, each "silent variation" of a nucleic acid which encodes a protein is implicit in each described sequence.
Furthermore, one of skill will recognize that individual substitutions deletions or additions that alter, add or delete a single amino acid or a small percentage of amino acids (typically less than 5%, more typically less than 1%) in an encoded sequence are "conservatively modified variations," where the alterations result in the substitution of an amino acid with a chemically similar amino acid. Conservative substitution tables providing functionally similar amino acids are well known in the art. The following five groups each contain amino acids that are conservative substitutions for one another: Aliphatic: Glycine (G), Alanine (A), Valine (V), Leucine (L), Isoleucine (I); Aromatic: Phenylalanine (F), Tyrosine (Y), Tryptophan (W); Sulfur-containing: Methionine (M), Cysteine (C); Basic: Arginine (R), Lysine (K), Histidine (H); Acidic: Aspartic acid (D), Glutamic acid (E), Asparagine (N), Glutamine (Q). *See also,* Creighton (1984) Proteins, W.H. Freeman and Company. In addition, individual substitutions, deletions or additions which alter, add or delete a single amino acid or a small percentage of amino acids in an encoded sequence are also "conservatively modified variations."

A "subsequence" refers to a sequence of nucleic acids or amino acids that comprise a part of a longer sequence of nucleic acids or amino acids (e.g., protein) respectively.
Nucleic acids are "elongated" when additional nucleotides (or other analogous molecules) are incorporated into the nucleic acid. Most commonly, this is performed with a polymerase (e.g., a DNA polymerase), e.g., a polymerase which adds sequences at the 3' terminus of the nucleic acid.
Two nucleic acids are "recombined" when sequences from each of the two nucleic acids are combined in a progeny nucleic acid. Two sequences are "directly" recombined when both of the nucleic acids are substrates for recombination. Two sequences are "indirectly recombined" when the sequences are recombined using an intermediate such as a cross-over oligonucleotide. For indirect recombination, no more than one of the sequences is an actual substrate for recombination, and in some cases, neither sequence is a substrate for recombination.
A "specific binding affinity" between two molecules, for example, a ligand and a receptor, means a preferential binding of one molecule for another in a mixture of molecules. The binding of the molecules can be considered specific if the binding affinity is about 1 x 10⁴ M⁻¹ to about 1 x 10⁶ M⁻¹ or greater.
Substrate: a substrate is the molecule that an enzyme naturally recognizes and converts to a product in the biochemical pathway in which the enzyme naturally carries out its function, or is a modified version of the molecule, which is also recognized by the enzyme and is converted by the enzyme to a product in an enzymatic reaction similar to the naturally-occurring reaction.
Transformation: a process for introducing heterologous DNA into a cell, tissue, or insect. Transformed cells, tissues, or insects are understood to encompass not only the end product of a transformation process, but also transgenic progeny thereof.
"Transformed," "transgenic," and "recombinant" refer to a host organism such as a bacterium or a plant into which a heterologous nucleic acid molecule has been introduced. The nucleic acid molecule can be stably integrated into the genome of the host or the nucleic acid molecule can also be present as an extrachromosomal molecule. Such an extrachromosomal molecule can be auto-replicating. Transformed cells, tissues, or plants are understood to encompass not only the end product of a transformation process, but also transgenic progeny thereof. A "non-transformed," "non-transgenic," or "non-recombinant" host refers to a wild-type organism, e.g., a bacterium or plant, which does not contain the heterologous nucleic acid molecule.

The present invention relates to transgenic hosts particularly, transgenic plants, plant tissues, plant seeds, and plant cells comprising a heterologous polynucleotide encoding a gene product where the gene product comprises trichothecene resistance activity and methods of making and using the same. Trichothecene resistance activity as used herein refers to an activity that reduces or inhibits the phytotoxicity of a trichothecene, particularly to a fungus and/or plant, in a particular embodiment of the invention trichothecene resistance activity refers to an activity that transfers an acetate to the C-3 position of a trichothecene.
The present invention further relates to transgenic hosts, particularly, transgenic plants, plant tissues, plant seeds, and plant cells expressing a heterologous polynucleotide encoding a gene product, the gene product having trichothecene resistance activity, particularly an acetyl transferase gene product, more particularly a 3-O-acetyl transferase gene product, more particularly trichothecene 3-O- acetyl transferase gene product and methods of making and using the same. Expression of the heterologous polynucleotide of the invention comprises the synthesis of RNA and may be detected by northern blot analysis. Particularly, expression of the heterologous polynucleotide of the invention may be detected where a labeled probe derived from a heterologous nucleotide of the invention, in particular embodiments, from SEQ ID NOs. 1, 5 or 7, hybridizes with RNA isolated from a transgenic plant of the invention in 7% sodium dodecyl sulfate (SDS), 0.5 M Sodium phosphate pH 7.0, 1 mM EDTA, 10 mg/ml BSA at 65°C with washing in .5% BSA (fraction V), 5% SDS, 40 mM Sodium phosphate pH 7.0, 1mM EDTA, .25 M sodium chloride at 65 ° C, preferably in 1% SDS, 40 mM Sodium phosphate pH 7.0, 1 mM EDTA, .125 M sodium chloride at 65 ° C, and preferably in 1% SDS, 40 mM Sodium phosphate pH 7.0, 1 mM EDTA at 65 ° C.
The present invention further relates to transgenic plants plant tissues, plant seeds, and plant cells, expressing a heterologous polynucleotide of the invention where the plant, plant cell, plant tissue or plant seed is trichothecene resistant. Trichothecene resistant plants, plant cells, plant tissues and plant seeds as used herein are those which are capable of metabolism in the presence of a trichothecene which may be determined as described in Example 7 below. In a particular embodiment, trichothecene resistant plants, plant tissues, plant cells and plant seeds which have a specific enzyme activity of at least 10 nmol triacetoxyscirpenol (hereinafter "TAS ")/microgram protein/15 min incubation at saturating substrate levels, more particularly at least 5 nmol TAS/microgram protein/15 min, more particularly at least 1 nmol TAS/microgram protein/15 min, more particularly at least 0.8 nmol TAS/microgram protein/15 min more particularly at least 0.5 nmol TAS/microgram protein/15 min, more particularly a specific activity of 0.25 nmol TAS/microgram protein/ 15 minute, more particularly a specific activity of 0.1 nmol TAS/microgram protein/15 min., more particularly a specific activity of 0.05 nmol TAS/microgram protein/15 min and even more particularly a specific activity of 0.01 nmol TAS/microgram protein/15 min above background levels of activity that occur naturally in a wild type control, particularly as determined in an assay as described in Example 6 below.
Trichothecene resistant plants of the invention comprise those of which a greater percentage of the seed germinate and form roots in the presence of a trichothecene than the seed from a wild type control where the trichothecene is present at a concentration of at least 5 microgram/ml, more preferably at least 10 microgram/ml, more preferably at least 15 microgram/ml, more preferably at least 20 microgram/ml and more preferably at least 25 microgram/ml. In a particularly preferred embodiment, trichothecene resistant plants of the invention comprise those of which at least 10% more seed, more preferably at least 20% more seed, more preferably at least 30% more seed, more preferably at least 40% more seed, more preferably at least 50% more seed, more preferably at least 60% more seed, more preferably at least 70% more seed, more preferably at least 80% more seed and more preferably at least 90% more seed germinate and form roots in the presence of a trichothecene than the seed of a wild type control.
Trichothecenes are frequently divided into several different structural groups. A particular embodiment of the present invention is drawn to resistance to group A and B trichothecenes. Groups A and B comprise the *Fusarium* trichothecenes and are differentiated primarily by the absence (group A) or presence (group B) of a carbonyl functional group at position C-8. The group B trichothecene DON, accordingly, comprises a carbonyl group at the C-8 position.
The present invention is more particularly drawn to resistance to trichothecenes, which contain a C-3 hydroxyl. Such trichothecenes include T-2 toxin, HT-2 toxin, isotrichodermol, DAS, 3-deacetylcalonectrin, 3,15-dideacetylcalonectrin, scirpentriol, neosolaniol; 15-acetyldeoxynivalenol, nivalenol, 4-acetylnivalenol (fusarenone-X), 4,15-diacetylnivalenol, 4,7,15-acetylnivalenol, and DON and their various acetylated derivatives.
In a particular embodiment, the trichothecene resistant plant, cell, tissue or seed thereof is resistant to a trichothecene producing fungus, particularly a fungus of the genus *Fusarium.* Fungus resistance as used herein refers to no initiation of infection after fungal inoculation or reduced spread of the infection after fungal inoculation compared to a wild type control.
In a preferred embodiment, a fungal resistant transgenic plant of the present invention is a cereal plant and under fungal challenge comprises less infected kernels or seeds compared to a wild type control, preferably at least a 10% decrease of infected kernels or seeds compared to the same number of kernels or seeds evaluated in a wild type control, more preferably at least a 20% decrease, more preferably at least a 40% decrease and more preferably at least a 50% decrease of infected kernels compared to the same number of kernels or seeds in a wild type control. The fungal resistant transgenic cereal plants of the invention comprise but are not limited to maize, wheat, barley, rice, and oats.
In wheat, fungal spread in the head may be evaluated as described in Example 9 below, by counting the number of symptomatic and asymptomatic spikelets on each inoculated head and calculating the percentage of spikelets on each head that are symptomatic. In a preferred embodiment, fungal resistant wheat of the present invention comprises, under fungal challenge, less infected spikelets than the wild type control, preferably at least a 10% decrease of infected spikelets compared to the same number of spikelets evaluated in a wild type control, more preferably at least a 20% decrease, more preferably at least a 40% decrease and more preferably at least a 50% decrease of infected spikelets compared to the same number of spikelets in a wild type control.
In maize, fungal spread in the ear may be evaluated by visual estimation of the percentage of infected kernels as described further in Example 9 below. In a preferred embodiment, fungal resistant maize of the invention, under fungal challenge, comprise less infected kernels than the wild type control, preferably at least a 10% decrease in infected kernels compared to the number of infected kernels in the same number of ears visibly estimated in a wild type control, more preferably at least a 20% decrease, more preferably at least 30% decrease, more preferably at least a 40 % decrease and more preferably at least a 50% decrease in infected kernels compared to the same number of ears visibly estimated in a wild type control. In maize, internal fungal spread in the stalk may be visually evaluated by splitting open the stalk and assessing the amount of discoloration. In a preferred embodiment of the invention, the transgenic maize of the invention comprises less internal and/or external discoloration of the stalk compared to a wild type control.
In another, preferred embodiment fungal resistant plants of the invention comprise those of which a greater percentage of seed germinate in the presence of fungal challenge than germinate in the wild type control. In a particularly preferred embodiment, fungal resistant plants of the invention comprise those of which at least 10% more seed, more preferably at least 20% more seed, more preferably at least 30% more seed, more preferably at least 40% more seed, more preferably at least 50% more seed, more preferably at least 60% more seed, more preferably at least 70% more seed, more preferably at least 80% more seed and more preferably at least 90% more seed, more preferably at least 100% more seed, more preferably at least 150% more seed germinates in the presence of *Fusarium* than does seed from the wild type control.
In another preferred embodiment, fungal resistant transgenic plants producing seed or kernels having less mycotoxin, e.g. trichothecene contamination, than the seed of a wild type control are provided. In a particularly preferred embodiment crop plants and more particularly cereal plants producing seed having at least 10% less trichothecene, more preferable at least 20% less trichothecene, more preferably at least 30% less trichothecene, more preferably at least 40% less trichothecene, more preferably at least 50% less trichothecene, more preferably at least 60% less trichothecene, more preferably at least 70% less trichothecene and more preferably at least 80% less trichothecene contamination than a wild type control are provided. Trichothecene contamination may be determined as described in Example10 below.
The polynucleotides of use in the invention include heterologous polynucleotides encodirig' acetyl transferases, particularly those encoding acetyl transferases capable of conferring trichothecene resistance, more particularly those encoding trichothecene 3-O-acetyltransferases. In a particular embodiment, the heterologous polynucleotide of the invention may be derived from but is not limited to fungal origin, more particularly from *Fusarium, Trichothecium,* and *Myrothecium* origin, more particularly from a *Fusarium* species such as *F. acuminatum, F. crookwellense, F. culmorum, F. equiseti, F. graminearum (Gibberella zeae), F. lateritium, F. poae, F. sambucinum (G. pulicaris),* and *F. sporotrichioides.* Heterologous polynucleotides of use in the invention include SEQ ID NO:1, 5 and/or 7 and sequences substantially similar to SEQ ID NO:1, 5 and/or 7.
A polynucleotide of use in the invention can be incorporated into host cells, such as plant, fungal or bacterial cells, using conventional recombinant DNA technology. Generally, this involves inserting the polynucleotide into an expression system to which the polynucleotide is heterologous using standard cloning procedures known in the art. The vector contains the necessary elements for the transcription and translation of the polynucleotide of use in the invention in a host cell containing the vector. A large number of vector systems known in the art can be used, such as plasmids, bacteriophage viruses and other modified viruses. The components of the expression system may also be modified to increase expression. For example, truncated sequences, nucleotide substitutions, nucleotide optimization or other modifications may be employed. Expression systems known in the art can be used to transform virtually any crop plant cell under suitable conditions. A heterologous polynucleotide of the inventions is preferably stabley transformed and integrated into the genome of the host cells. In another preferred embodiment, the heterologous polynucleotide of the inventions is located on a self-replicating vector. Examples of self-replicating vectors are viruses, in particular Gemini viruses. Transformed cells can be regenerated into whole plants such that the chosen form of the polynucleotide of the invention confers trichothecene resistance in the transgenic plants.

A polynucleotide of the invention intended for expression in transgenic plants is first assembled in an expression cassette behind a suitable promoter expressible in plants. The expression cassettes may also comprise any further sequences required or selected for the expression of the heterologous polynucleotide of the invention. Such sequences include, but are not restricted to, transcription terminators, extraneous sequences to enhance expression such as introns, vital sequences, and sequences intended for the targeting of the gene product to specific organelles and cell compartments. These expression cassettes can then be easily transferred to the plant transformation vectors described *infra.* The following is a description of various components of typical expression cassettes.

The selection of the promoter used in expression cassettes will determine the spatial and temporal expression pattern of the heterologous polynucleotide of the invention in the transformed plant. Selected promoters will express heterologous polynucleotides of the invention in specific cell types (such as leaf epidermal cells, mesophyll cells, root cortex cells) or in specific tissues or organs (roots, leaves or flowers, for example) and the selection will reflect the desired location of accumulation of the gene product. Alternatively, the selected promoter may drive expression of the gene under various inducing conditions. Promoters vary in their strength, i.e., ability to promote transcription. Depending upon the host cell system utilized, any one of a number of suitable promoters known in the art can be used. For example, for constitutive expression, the CaMV 35S promoter, the rice actin promoter, or the ubiquitin promoter may be used. For regulatable expression, the chemically inducible PR-1 promoter from tobacco or *Arabidopsis* may be used (*see, e.g.,* U.S. Patent No. 5,689,044).

A variety of transcriptional terminators are available for use in expression cassettes. These are responsible for the termination of transcription beyond the heterologous polynucleotide of the invention and its correct polyadenylation. Appropriate transcriptional terminators are those that are known to function in plants and include the CaMV 35S terminator, the *tml* terminator, the nopaline synthase terminator and the pea *rbcS* E9 terminator. These can be used in both monocotyledonous and dicotyledonous plants.

Numerous sequences have been found to enhance gene expression from within the transcriptional unit and these sequences can be used in conjunction with the polynucleotides of this invention to increase their expression in transgenic plants. For example, various intron sequences such as introns of the maize *Adhl* gene have been shown to enhance expression, particularly in monocotyledonous cells. In addition, a number of non-translated leader sequences derived from viruses are also known to enhance expression, and these are particularly effective in dicotyledonous cells.

The coding sequence of the selected gene optionally is genetically engineered by altering the coding sequence for optimal expression in the crop species of interest. Methods for modifying coding sequences to achieve optimal expression in a particular crop species are well known (see, e.g. Perlak et al., Proc.. Natl. Acad. Sci. USA 88: 3324 (1991); and Koziel et al., Bio/technol. 11: 194 (1993); Fennoy and Bailey-Serres. Nucl. Acids Res. 21: 5294-5300 (1993). Methods for modifying coding sequences by taking into account codon usage in plant genes and in higher plants, green algae, and cyanobacteria are well known (see table 4 in: Murray et al. Nucl. Acids Res. 17: 477-498 (1989); Campbell and Gowri Plant Physiol. 92: 1-11 (1990).

Various mechanisms for targeting gene products are known to exist in plants and the sequences controlling the functioning of these mechanisms have been characterized in some detail. For example, the targeting of gene products to the chloroplast is controlled by a signal sequence found at the amino terminal end of various proteins which is cleaved during chloroplast import to yield the mature protein (*e.g.* Comai et al. J. Biol. Chem. 263: 15104-15109 (1988)). Other gene products are localized to other organelles such as the mitochondrion and the peroxisome (*e.g.* Unger et al. Plant Molec. Biol. 13: 411-418 (1989)). The cDNAs encoding these products can also be manipulated to effect the targeting of heterologous products encoded by DNA sequences to these organelles. In addition, sequences have been characterized which cause the targeting of products encoded by DNA sequences to other cell compartments. Amino terminal sequences are responsible for targeting to the ER, the apoplast, and extracellular secretion from aleurone cells (Koehler & Ho, Plant Cell 2: 769-783 (1990)). Additionally, amino terminal sequences in conjunction with carboxy terminal sequences are responsible for vacuolar targeting of gene products (Shinshi et al. Plant Molec. Biol. 14: 357-368 (1990)). By the fusion of the appropriate targeting sequences described above to a heterologous polynucleotide of the invention, it is possible to direct a resulting product to any organelle or cell compartment.

Numerous transformation vectors available for plant transformation are known to those of ordinary skill in the plant transformation arts, and the polynucleotides pertinent to this invention can be used in conjunction with any such vectors. The selection of vector will depend upon the preferred transformation technique and the target species for transformation. For certain target species, different selection markers may be preferred. Selection markers used routinely in transformation include the *nptll* gene, which confers resistance to kanamycin and related antibiotics (Messing & Vierra. Gene 19: 259-268 (1982); Bevan et al., Nature 304:184-187 (1983)), the *bar* gene, which confers resistance to the herbicide phosphinothricin (White et al., Nucl. Acids Res 18: 1062 (1990), Spencer et al. Theor. Appl. Genet 79: 625-631 (1990)), the *hph* gene, which confers resistance to the antibiotic hygromycin (Blochinger & Diggelmann, Mol Cell Biol 4: 2929-2931), and the *dhfr* gene, which confers resistance to methotrexate (Bourouis et al., EMBO J. 2(7): 1099-1104 (1983)), and the EPSPS gene, which confers resistance to glyphosate (U.S. Patent Nos. 4,940,935 and 5,188,642), phosphomannose isomerase gene, *manA,* which confers a selective metabolic advantage in the presence of mannose (U.S. Pat. Ser. No. 5,767,378 and Miles& Guest, GENE, 32:41-48 (1984)). PAT selectable marker that confers resistance to BASTA (Sung H. Park et al., In Vitro Cell.Dev.Biol.-Plant, 34: 117-121 (1998)).

Many vectors are available for transformation using *Agrobacterium tumefaciens.* These typically carry at least one T-DNA border sequence and include vectors such as pBIN19 (Bevan, Nucl. Acids Res. (1984)). Typical vectors suitable for *Agrobacterium* transformation include the binary vectors pCIB200 and pCIB2001, as well as the binary vector pCIB10 and hygromycin selection derivatives thereof. *(See,* for example, U.S. Patent No. 5,639,949).

Transformation without the use of *Agrobacterium tumefaciens* circumvents the requirement for T-DNA sequences in the chosen transformation vector and consequently vectors lacking these sequences can be utilized in addition to vectors such as the ones described above which contain T-DNA sequences. Transformation techniques that do not rely on *Agrobacterium* include transformation via particle bombardment, protoplast uptake (e.g. PEG and electroporation) and microinjection. The choice of vector depends largely on the preferred selection for the species being transformed. Typical vectors suitable for non-*Agrobacterium* transformation include pCIB3064, pSOG19, and pSOG35. (*See,* for example, U.S. Patent No. 5,639,949).

Once the polynucleotide of interest has been cloned into an expression system, it is transformed into a plant cell. Methods for transformation and regeneration of plants are well known in the art. For example, Ti plasmid vectors have been utilized for the delivery of foreign DNA, as well as direct DNA uptake, liposomes, electroporation, micro-injection, and microprojectiles. In addition, bacteria from the genus *Agrobacterium* can be utilized to transform plant cells.
Transformation techniques for dicotyledons are well known in the art and include *Agrobacterium*-based techniques and techniques that do not require *Agrobacterium.* Non-*Agrobacterium* techniques involve the uptake of exogenous genetic material directly by protoplasts or cells. This can be accomplished by PEG or electroporation mediated uptake, particle bombardment-mediated delivery, or microinjection. In each case the transformed cells are regenerated to whole plants using standard techniques known in the art.
Transformation of most monocotyledon species has now also become routine. Preferred techniques include direct gene transfer into protoplasts using PEG or electroporation techniques, particle bombardment into callus tissue, as well as Agrobacterium-mediated transformation. Target tissue may be derived from such sources as wheat cultivar UC703 or maize genotype CG000526. For example, *Agrobacterium* mediated transformation of maize may be carried out as described in U.S. patent application 09/089,111 correspondingly published as WO 98/54961, and of barley may be carried out as described by: M. Cho, J. Wong, C. Marx, W. Jiang, P. Lemaux and B. Buchanan (1999). Overexpression of thioredoxin h leads to enhanced activity of starch debranching enzyme (pullulanase) in barley grain. PNAS 96: 14641-14646; S. Zhang, M. Cho, T. Koprek, R. Yun, P. Bregitzer and P. Lemaux (1999). Genetic transformation of commercial cultivars of oat (Avena sativa L.) and barley (Hordeum vulgare L.) using in vitro shoot meristematic cultures derived from germinated seedlings. Plant Cell Rep. 18: 959-966; P. Bregitzer, S. Harlbert and P. Lemaux (1998). Somaclonal variation in the progeny of transgenic barley. TAG 96: 421-425; M. Cho, W. Jiang and p. Lemaux (1998). Transformation of recalcitrant barley cultivars through improvement of regenerability and decreased albinism. Plant sci. 138: 229-244; P. Lemaux, m. Cho, S. Zhang, and p. Bregitzer (1998). Transgenic cereals: Hordeum vulgare L. - current status and future prospects. In: Vasil I, Phillips R (eds) Molecular Improvement of Cereal Crops, Kluwer Academic Publ, Dordrecht, The Netherlands, pp 255-316; S. Zhang, R. Williams-Carrier, D. Jackson, and P. Lemaux (1998). Expression of CDC2Zm and KNOTTED1 during in vitro aaxillary shoot meristem proliferation and adventitious shoot meristem formation in maize (Zea mays L.) and barley (Hordeum vulgare L.). Planta 204: 542-549; D. McElroy, J. Louwerse, S. McElroy and P. Lemaux (1997). Development of a simple transient assay for Ac/Ds activity in cells of intact barley tissue. Plant J. 11: 157-165; S. Tingay, D. McElroy, R. Kalla, S. Fieg, M. Wang, S. Thornton and R. Brettell (1997). Agrobacterium tumefaciens-mediated bareley transformation. The Plant J. 11: 1369-1376; J.Qureshi, Z. Basri, R. Singh, R. Burton, M. Dalton, J. Kollmorgen and G. Fincher. 1988. Agrobacterium-mediated transformation of two varieties of barley (Hordeum vulgare L.) Proc. 42nd. Conference of Australian Society for Biochemistry and Molecular Biology, September 28-October 1, 1998, Adelaide, Australia; J. Qureshi, R. Singh, Z. Basri, R. Stewart, R. Burton, J. kollmorgen and G. Fincher (1997). Strategies for genetic transformation of elite Australian barley varieties. Proc. 8th. Aust.Barley Technical symp. Gold Coast, Queensland, 7-12 September 1997. 2:8.9-11; P. Lemaux, M. Cho, J. Louwerse, R. Williams and Y. Wan (1996). Bombardment-mediated transformation methods for barley. Bio-Rad US/EG Bull 2007: 1-6; T. Koprek, R. Hansch, A. Nerlich, R. Mendel and J. Schulze (1996). Fertile transgenic barley of different cultivars obtained by adjustment of bombardment conditions to tissue response. Plant Sci. 119: 79-91; T. Hagio, T. hirabayashi, H. Machii and H. Tomutsune (1995). Production of fertile transgenic barley (Hordeum vulgare L.) plants using the hygromycin-resistance marker. Plant Cell Rep. 14: 329-334; H. Funatsuki, H. Kuroda, M. Kihara, P. Lazzeri, E. Muller, H. Lorz and I. Kishinami (1995). Fertile transgenic barley regenerated by direct DNA transfer to protoplasts. TAG 91: 707-712; A. Jahne, D. Becker, R. Brettschneider and H. Lorz (1994). Regeneration of transgenic, microscpore-derived, fertile barey. TAG 89: 525-533; Y. Wan and P. Lemaux (1994). Generation of large numbers of independently transformed fertile barley plants. Plant Physiol. 104: 37-48.

The polynucleotides of the invention can be utilized to confer trichothecene resistance to a wide variety of plant cells, including those of gymnosperms, monocots, and dicots. Although the heterologous polynucletide of the invention can be inserted, e.g. transformed into any plant cell falling within these broad classes, it is particularly useful in crop plant cells, such as rice, wheat, barley, rye, corn, oats, potato, sweet potato, turnip, squash, pumpkin, zucchini, melon, soybean, and sorghum. The polynucleotides of use in the invention rendering a plant trichothecene resistant may be used in combination with other characteristics important for production and quality. The polynucleotides of the invention can be incorporated into plant lines through breeding approaches and techniques known in the art. Where a trichothecene resistant gene allele is obtained by transformation into a crop plant or plant cell culture from which a crop plant can be regenerated, it is moved into commercial varieties using traditional breeding techniques to develop a trichothecene resistant crop without the need for genetically engineering the allele and transforming it into the plant.
The various breeding steps are characterized by well-defined human intervention such as selecting the lines to be crossed, directing pollination of the parental lines, or selecting appropriate descendant plants. Depending on the desired properties different breeding measures are taken. The relevant techniques are well known in the art and include but are not limited to hybridization, inbreeding, backcross breeding, multiline breeding, variety blend, interspecific hybridization, aneuploid techniques, etc.. Hybridization techniques also include the sterilization of plants to yield male or female sterile plants by mechanical, chemical or biochemical means. Cross pollination of a male sterile plant with pollen of a different line assures that the genome of the male sterile but female fertile plant will uniformly obtain properties of both parental lines. Thus, the transgenic seeds and plants according to the invention can be used for the breeding of improved plant lines exhibiting no or reduced fungal growth on the plant, plant tissue, or seed thereby preventing and/or reducing mycotoxin contamination of said plant, plant tissue or seed.

The trichothecene resistance engineered into the transgenic seeds and plants mentioned above are passed on by sexual reproduction or vegetative growth and can thus be maintained and propagated in descendant plants. Generally said maintenance and propagation make use of known agricultural methods developed to fit specific purposes such as tilling, sowing or harvesting. As the growing crop is vulnerable to attack and damages caused by insects or infections, measures are undertaken to control plant diseases, insects, nematodes, and other adverse conditions to improve yield. These include mechanical measures such a tillage of the soil or removal of infected plants, as well as the application of agrochemicals such as fungicides, gametocides, nematicides, growth regulants, ripening agents and insecticides.
In seeds production germination quality and uniformity of seeds are essential product characteristics, whereas germination quality and uniformity of seeds harvested and sold by the farmer is not important. As it is difficult to keep a crop free from other crop and weed seeds, to control seedbome diseases, and to produce seed with good germination, fairly extensive and well-defined seed production practices have been developed by seed producers, who are experienced in the art of growing, conditioning and marketing of pure seed. Thus, it is common practice for the farmer to buy certified seed meeting specific quality standards instead of using seed harvested from his own crop. Propagation material to be used as seeds is customarily treated with a protectant coating comprising herbicides, insecticides, fungicides, bactericides, nematicides, molluscicides or mixtures thereof. Customarily used protectant coatings comprise compounds such as captan, carboxin, thiram (TMTD^{®}), methalaxyl (Apron^{®}), and pirimiphos-methyl (Actellic^{®}). If desired these compounds are formulated together with further carriers, surfactants or application-promoting adjuvants customarily employed in the art of formulation to provide protection against damage caused by bacterial, fungal or animal pests. The protectant coatings may be applied by impregnating propagation material with a liquid formulation or by coating with a combined wet or dry formulation. Other methods of application are also possible such as treatment directed at the buds or the fruit.

It is a further aspect of the present invention to provide new agricultural methods such as the methods examplified above which are characterized by the use of transgenic plants, transgenic plant material, or transgenic seed according to the present invention.

In another embodiment, the present invention relates to a transgenic plant cell, tissue, organ, seed or plant parts obtained from the transgenic plant. Also included within the invention are transgenic descendants of the plant as well as transgenic plant cells, tissues, organs, seeds and plant parts obtained from the descendants.

In another embodiment, the heterologous polynucleotide of use in the invention, can also be used as a selectable marker in transformation procedures. In this aspect the host cell is transformed with a second heterologous polynucleotide of interest as well as a heterologous polynucleotide of the invention which encodes a gene product comprising trichothecene resistance activity, using expressions cassettes and transformation techniques exemplified above and known in the art. After transformation, the transformed cells are selected for their ability to survive when exposed to a trichothecene, particularly DAS or DON or T-2 toxin. The host cell may be a eukaryotic or prokaryotic host cell using transformation and expression systems known in the art. The host cell may be a plant cell, a fungal cell, a bacterial cell, a yeast cell, an animal cell, or an insect cell.

In a particularly preferred embodiment of the invention, a polynucleotide which encodes a gene product comprising trichothecene resistance activity is used as a selectable marker in plant cell transformation methods. For example, plants, plant tissue, plant seeds, or plant cells expressing at least a second heterologous DNA sequence of interest can also be transformed to express a sequence encoding a polypeptide comprising a sequence substantially similar to that of SEQ ID NO:2, 6 or 8. The transformed cells are transferred to medium containing a phytotoxic trichothecene, particularly DAS and/or DON and/or T-2 toxin, in an amount sufficient to inhibit the growth or survivability of plant cells not expressing the polypeptide substantially similar to that having the amino acid sequence of SEQ ID NO:2, 6 or 8, wherein only the transformed cells will grow or will be unstunted. Concentrations of trichothecenes useful for selection of plants expressing the polypeptide substantially similar to that having the amino acid sequence of SEQ ID NO:2, 6 or 8 range from 1 µg/ml to 90 µg/ml The method is applicable to any plant cell capable of expressing a polynucleotide comprising a nucleotide sequence substantially similar to that of SEQ ID NO: 1, 5 or 7, and can be used with any heterologous DNA sequence of interest. Expression of the second heterologous DNA sequence and the heterologus polynucleotide of the of the invention can be driven by the same promoter functional in plant cells, or by separate promoters.

### Description of the Sequences:

- SEQ ID NO: 1: is a cDNA sequence from *Fusarium sporotrichioides* encoding a polypeptide of the invention having trichothecene resistance activity.
- SEQ ID NO:2: is the polypeptide having trichothecene resistance activity encoded by SEQ ID NO:1.
- SEQ ID NO: 3: is a DNA primer.
- SEQ ID NO 4:: is a DNA primer.
- SEQ ID NO: 5: is a DNA sequence from *Fusarium graminearum* encoding a polypeptide of the invention having trichothecene resistance activity.
- SEQ ID NO: 6: is the polypeptide having trichothecene resistance activity encoded by SEQ ID NO. 3.
- SEQ ID NO. 7: is a DNA sequence from *Saccharomyces cerevisiae* encoding a polypeptide of the invention having trichothecene resistance activity.
- SEQ ID NO. 8: is the polypeptide having trichothecene resistance activity encoded by SEQ ID NO. 5.
- SEQ ID NO. 9: is the DNA sequence of pCIB9818.
- SEQ ID NO. 10: is the DNA sequence of pAgroTRlr.
- SEQ ID NO. 11: is the DNA sequence of pNOV1704.

### Examples

The following examples further describe the materials and methods used in carrying out the invention and the subsequent results. They are offered by way of illustration, and their recitation should not be considered as a limitation of the claimed invention.

### Example 1: Composition of pNOV1700, pCIB9818, pAgroTRlr and pNov 1704

### 1. pNOV1700:

pNOV1700 was deposited under the terms of the Budapest Treaty on March 19, 1999, with the Agricultural Research Service, Patent Culture Collection (NRRL), Northern Regional Research Center, 1815 Northern University Street, Peoria, Illinois 61604, USA and assigned accession number NRRL B-30117.

Accordingly, pNOV1700 comprises SEQ ID NO. 1 operably linked to the *Z.mays* ubiquitin promoter, including a portion of the exon and intron, and to the nopaline synthase polyadenylation signal.

### 2. pCIB9818

Plasmid pCIB9818 is a 6111 base pair circular plasmid having a DNA sequence according to SEQ ID NO. 9. The *Z. mays* ubiquitin promoter, base 12 to 1993 of SEQ ID NO.9, including a portion of the exon, base 896 to 1011, and the intron, base 1047 to 1993, is operably linked to the phosphate mannose isomorase selectable marker, base pair 2090 to 3193, the inverted PEPC intron #9 from base 3248 to 3355 and the termination sequence of the CaMV 35S gene, base 3357 to 3434.

### 3. pAgroTRlr

Plasmid pAgroTRlr is a 13,737 base pair circular binary vector having a DNA sequence according to SEQ ID NO. 10. Accordingly, pAgroTRlr comprises a selectable marker operably linked to a promoter and termination sequence and the polynucleotide region of SEQ ID NO: 1 behind and in frame with the *Arabidopsis thaliana* UBI 3 promoter (S. Norris, S. Meyer, and J. Callus, Plant Molecular Biology 22:895-906, (1993)) and in front of and in frame with the nos polyadenylation signal.

### 4. pNov1704

Plasmid pNOV1704 is a 12949 base pair circular binary vector having a DNA sequence according to SEQ ID NO. 11. The *Z. mays* ubiquitin promoter, base 11 to 1992 of SEQ ID NO.11 (including exon 1: 895 to 1010 and intron 1: 1046 to 1992), is operably linked to the phosphate mannose isomorase selectable marker sequence, base 2089 to 3192, and the nopaline synthase termination sequence, base 3557 to 3688. pNOV1704, further comprises the *Z. mays* ubiquitin promoter, base 9218 to 11218 (including exon 1: 10110 to 10224 and intron 1: 10225 to 11218) operably linked to the trichothecene 3-*O*-acetyl transferase sequence of SEQ ID NO.1, at base 11234 to 12662 and the nos termination sequence 12667 to 12935.

### Example 2: Wheat transformation, selection and regeneration

### Transformation

Immature zygotic embryos (0.75-1.25mm) are dissected from surface sterilized wheat caryopses (10% Chlorox X 10 minutes) then plated scutellum up onto an MS based medium (Murashige and Skoog, (1962) Physiol. Plant 15:473-439) supplemented with 3% sucrose, 3mg/liter 2,4-D (dichlororpheoxyacetic acid), 150mg/l glutamine, 75mg/l asparagine and solidified with 0.7% phytagar (3MS3S medium). The embryos are incubated in the dark at 28°C for 5-10 days prior to bombardment. The optimal time for bombardment is 6-7 days post-plating. Four hours prior to bombardment, the embryos are placed on a plasmolysis medium (same medium described above but with 15% maltose added in place of the sucrose) and arranged in a 2.5cm diameter circle with scutellum facing up.
pNOV1700, described in Example 1 above, is digested with Pvull and Xmnl and a 4117bp fragment comprising a polynucleotide region having a sequence according to SEQ ID NO:1 as well as the ubiquitin promoter and NOS polyadenylation signal is isolated. pCIB9818, also described in Example 1 above, is digested with Ascl and the 4246 bp fragment comprising the UBI maize promoter, selectable marker and CaMV 35S termination sequence is isolated.
The isolated DNA fragments are precipitated onto 0.3 micro meter gold particles using the standard Sanford method. While continuously vortexing , 5 microgram of the isolated fragment DNA per construct , 50ul of 2.5 M CaCl₂ and 20ul of 0.1 M spermidine are added to an eppendorf tube that contains 50ul of 50% glycerol and 3mg gold. The DNA/gold mixture is given two ethanol washes. After discarding the supernatant from the last wash, ethanol is added to make a final volume of 70ul. This provides six shots per tube of gold/DNA. The target plates are shot twice so that gives a delivery of approximately 3microgram DNA (if 5microgram each of 2 constructs is used which is usually the case) and 1.0 mg gold per target plate. The rupture pressure used is 7.584 MPa (1100 psi). After bombardment, the target plates are returned to the dark overnight. After approximately 24 hours of plasmolysis, the embryos are removed to 3MS3S and returned to the dark for 3 weeks of callus initiation. No subculturing is done during this time.

### Selection/Regeneration

The embryogenic tissue that develops during the 3-week initiation period is dissected away from non-embryogenic tissue and placed on a regeneration/selection medium. The basic regeneration medium is 3MS3S without the 2,4-D but with 1 mg/I GA3 (Gibberellin A₃) NAA (1-Naphtheleneacetic Acid) and NAA added instead (called NG medium). 10g/l mannose and 5g/l sucrose is added (NG1 M.5S). The tissue is subjected to this initial phase of regeneration and selection for 2 weeks. For most of the 2 week period the tissue is in the light room. Shoot and root development begins during this phase and after 2 weeks all tissue is taken to the next stage.

For the second phase of regeneration and selection with mannose selection, mannose is decreased to 5g/l and the sucrose increased to 20g/l (MS2S.5M). The tissue normally stays on these media for approximately 4 weeks time during which further shoot and root development occurs.

Vigorously growing plantlets with good color, and root and shoot development are removed from plates and placed in larger containers called GA7's. This is the final stage of selection and regeneration The medium contains only 1/2MS salts and 15g/l mannose. The best indicator that a plant may be transformed is the observance of active root growth into the medium. Leaf tissue from actively growing plantlets is collected and PCR is done for either the gene of interest or selectable marker before transferring to the green house.

### EXAMPLE 3: Arabidopsis transformation

The binary vector pAgroTRlr constructs described in Example 1 above is transformed into *Agrobacterium tumefaciens* strain GV3101 (Bechtold, N. et al., CR Acad. Sci. Paris, Sciences de la vie, 316:1194-1199 (1993)) by electroporation (Dower, W.J., Mol. Biol. Rep 1:5 (1987) A 25 ml culture from single colonies of GV3101 agrobacterium containing pAgroTRlr plasmids in YEB + Rifampicin 100 and kanamycin 100 is incubated at 30 degrees overnight. Large cultures are started by inoculating 500 ml of the same media with 5 mls of the small culture and are incubated overnight at 30 degrees. The OD at 600 nm of cultures is determined and the cultures are then spun down at 5 K in the GSA rotor for 15 minutes. Cells are resuspended in "IM Modified infiltration media" to achieve a final O. D. at 600 nm of .08. 200 microliters of Silwet per liter of suspended cells is added. Three pots of bolting Arabadopsis var Columbia about 4 plants per pot, are inverted in about 500 ml of cell suspension. The flowers are shaken in the cell suspension to dislodge the air bubbles and the plants are incubated in the cell suspension for 15 minutes. A dome is placed on the tray to keep the plants humid overnight.
Plants are allowed to grow about 3-4 weeks after which the plants are not watered for up to 1 week. Seed pods are collected and dried in drying room for about a week and a half. The seeds are planted and allowed to grow for about 2 weeks. The plants are sprayed with the selection agent and then sprayed again 2 days later and 4 days later. After about three days surviving plants can be transplanted to new pots.

### EXAMPLE 4: Maize biolistic transformation.

Type I embryogenic callus cultures (Green et al 1983, Somatic cell genetic systems in corn. A. Fazelahmad, K. Downey, J. Schultz, RW Voellmy, eds. Advances in Gene Technology: Molecular Genetics of Plants and Animals. Miami Winter Symposium Series, Vol. 20. Academic Press, NY.) are initiated from immature maize embryos, that are 1.5 - 2.0 mm in length, from greenhouse grown material. Embryos are aseptically excised from surface-sterilized ears approximately 14 days after pollination. The embryos are placed on D callus initiation media (Duncan et al,(1985) Planta 165:pp322-332) with 2% sucrose and 5mg/L chloramben. Embryos and embryogenic cultures are subsequently cultured in the dark. Embryogenic responses are excised from the explants after about 14 days. Responses are placed onto D callus maintenance media with 2% sucrose and 0.5mg/L 2,4-D. After about 6 weeks of weekly selective subculture to fresh maintenance media, high quality compact embryogenic cultures are established. Actively growing embryogenic callus pieces are selected as target tissue for gene delivery. The callus pieces are plated onto target plates containing maintenance medium with 12% sucrose approximately 4 hours prior to gene delivery.
The callus pieces are arranged in circles, with radii of 8 and 10mm from the center of the target plate.
pNOV1700, described in Example 1 above, is digested with Pvull and Xmnl and a 4117bp fragment comprising a polynucleotide region having a sequence according to SEQ ID NO:1 isolated as well as promoter and polyadenylatin signal. pCIB9818, also described in Example 1 above, is digested with Ascl and the 4246 bp fragment comprising the marker gene, promoter and termination signal is isolated. The isolated DNA fragments are precipitated onto gold microcarriers as described in the DuPont Biolistics manual. Two to three µg for each plasmid construct is used in each 6 shot microcarrier preparation. Polynucleotides of the invention are delivered to the target tissue cells using the PDS-1000He Biolistics device. The settings on the Biolistics device are as follows: 8 mm between the rupture disk and the macrocarrier, 10 mm between the macrocarrier and the stopping screen and 7 cm between the stopping screen and the target. Each target plate is shot twice using 4.482 (650psi) rupture disks. A 200 X 200 stainless steel mesh (McMaster-Carr, New Brunswick, NJ) is placed between the stopping screen and the target tissue. Seven days after gene delivery, target tissue pieces are transferred from the high osmotic medium to selection medium.
The target tissue is placed onto maintenance medium containing no sucrose and 1% mannose. After 3 to 5 weeks, growing callus pieces are subcultured to the maintenance medium containing no sucrose and 1.5% mannose. Embryogenic callus growing on selection media is subcultured every 2 weeks for 6 to 10 weeks until enough callus is produced to generate 10-20 plants. Tissue surviving selection from an original target tissue piece is subcultured as a single colony and designated as an independent transformation event. Colonies are transferred to a modified MS medium (Murashige and Skoog, 1962(1962) A revised medium for rapid growth and bioassays with tobacco tissue cultures. Physiol. Plant 15: 473-497.) containing 2% sucrose and 1% mannose (MS2S+1 M) with 0.25mg/L ancymidol and 0.5mg/L kinetin. After 2 weeks, regenerating colonies are then transferred to MS2S+1 M without hormones. Regenerating shoots with or without roots from all colonies are transferred to Magenta boxes containing MS3S medium and small plants with roots are recovered and transferred to soil in the greenhouse.

### EXAMPLE 5: Analyses of transgenic plant expression

Tissue from transformed plants is analyzed for the presence of a polynucleotide comprising the sequence of SEQ ID NO:1. DNA is extracted from transformed plant and PCR analyses are performed according to standard protocols. The primers used for amplification of the gene constructs are (5'-acgaatcattcaccgaggag-3') (SEQ ID No. 3) and (5'-ctcacactctcaggcttacc-3') (SEQ ID NO. 4). A 650 nt fragment within the sequence of SEQ ID NO:1 in wheat obtained according to Example 2 above is detected.

### b. Northern analysis

Transformed plants are analyzed for the presence of RNA by northern blot hybridization. For northern blot analysis, RNA extracted from plant tissue is size separated and blotted onto a nylon membrane. This membrane is subsequently hybridized with a radioactive probe, derived from the 429 nt Styl fragment of the polynucleotide according to SEQ ID NO:1 is used as the probe. RNA is detected in wheat and Arabidopsis plants transformed according to examples 2 and 3 above.

### EXAMPLE 6: Enzymatic assay for trichothecene 3-O-acetyltransferase activity.

*a.) Extraction of plant tissue for enzyme assays:* Three 1 x 1/8 in pieces of leaf (about 50 mg) from transgenic plants of the invention including those transformed and regenerated according to Examples 2-4 above are selected.
*(b) Glass Bead Mill:* Tissue is placed in 2 ml round bottomed tube and the cap closed. The tube is immersed in liquid nitrogen and is incubated overnight at -80 °C. Tube is shaken on saws-all 24 seconds and 0.4 ml sodium phosphate buffer is added. The tube is vortexed about 10 seconds and is placed on ice. The tube is vortexed another 5 minutes and then is spun at 14,000 rpm in Eppendorf centrifuge 5 min. The supernatant is removed and is placed in a clean tube.
The following components are mixed
**trichothecene substrate**, 2 microliters of DAS (20% acetone in 50 mM Sodium phosphate buffer pH 7.0). DON may also be used.
**Acetyl CoA substrate,** 2 micro liters of [¹⁴C]-acetyl CoA NEN cat. # NEC313 (60 mCi/millimole and 0.02 mCi/ml)
Buffer, to a final volume of 50 µl with sodium phospahate buffer pH 7.0

The assay is initiated by adding the following enzyme preparation and is incubated at 30 °C for 15 minutes.

**Enzyme preparation,** 10 microliter plant extract in sodium phosphate buffer pH 7.0

After 15 minutes, 100 microliters ethyl acetate is added and the tube is vortexed twice for several seconds. The tube is spun for 2 minutes at 14,000 rpm in an Eppendorf centrifuge. 50 microliters of the ethyl acetate phase is removed and is added to a vial containing scintillation cocktail. The tube is counted for 2 min. using a scintillation counter.

Twenty separate wheat plants obtained according to Example 2 above and having specific activities of 0.60 to 13.4 nmol acetylated product/µg protein/15 min are identified. The specific activities of the transformed wheat plants are significantly greater than the negative control. The negative control is a non-transformed wheat cultivar, which has a specific activity of 0.1 to 0.2 nmol acetylated product/µg protein/15 min.

Five separate Arabidopsis plants obtained according to Example 3 above and having specific activities ranging from 3.8 to 28 nmol acetylated product/µg protein/15 min are identified. The specific activities of the transformed plants are significantly greater than that of the negative control. The negative control is an *Arabidopsis thaliana* var Columbia transformed with a nucleic acid construct for expressing the selectable marker which has a specific activity of less than 0.1 nmol acetylated product/µg protein/15 min.

*Maize* plants from at least two different transformants obtained according to Example 4 above and having specific activities ranging from 11.1 to 17.9 nmol acetylated product/µg protein/15 min are identified. The specific activities of the transformed plants are significantly greater than that of the negative control. The negative control is a non-transformed *maize* genotype that has a specific activity of less than 0.2 nmol acetylated product/µg protein/15 min.

Maize plants from at least 16 different transformants obtained using *Agrobacterium* mediated transformation of pNOV 1704, having specific activities ranging from 17 to 183 nmol/microgram/15 min are identified.

### EXAMPLE 7: Bioassay for trichothecene resistance in transgenic plants.

250 ml of CPR media having the following components is prepared and the pH adjusted to 6.5 with KOH.

| | |
|---|---|
| ½ MS salts | 0.54 g |
| ½ MS vitamins | 1.25 ml |
| Sucrose 1% (optional) | 2.50 g |

Agarose is added to the above media to a concentration of 1% (2.50 g) and the media is autoclaved. 25 ml of 50 mg/ml chlorophenol red is added to the autoclaved media.

While media is maintained at 55 °C, DAS or DON is added in acetone at various concentrations. (i. e. DON at 4, 8, or 16 microliters 10 mg/ml or DAS at 2, 4, or 6 microliters 50 mg/ml DAS per 1.7 ml). About 0.5 ml of medium is aliquoted to each well in a 48 well microtiter plate.

3.467 mm x 3.175 mm (1/3 x 1/8 inch) pieces of transformed plant tissue are added to microtiter plate wells as well as control tissue from untransformed wild type controls. The leaf pieces are allowed to fall into a petri dish and are pushed into the microtiter plate well media with tweezers. The microtiter plates are incubated 2 to 4 days at 20 °C under lights. Leaf piece metabolism results in color change (drop in pH) from red to yellow. Trichothecene resistance activity or reduced sensitivity to trichothecenes by transformants, results in yellow colored wells in the presence of DAS or DON.

A color change from red to yellow compared to the control that remains red is observed in wheat and maize plants of the invention. Furthermore, the individual leaf pieces have significantly less chlorosis than the corresponding control.

### EXAMPLE 8: Germination Assay

### A. Trichothecene Resistance Germination Assay

Seed from transgenic plants of the invention is grown under selective pressure from the selection agent and the resulting plants are selfed. The resulting seed is plated on MS3S medium (MS salts 4.3 g/L, MS vitamins 100X, Sucrose 30 g/L, and phytagar 8 g/L) and supplemented with either DAS or DON (at 20 mg/ml) at a density of 1000 to 1200 seeds/petri dish (100 mm diameter). After incubation in the light for four days the plates are examined for seedling growth.
Arabidopsis seed from plants obtained according to Example 3 above and grown in media comprising DAS, has numerous plants with both root and shoot development. While control seed (parental Arabadopsis line, var.Columbia) germinates poorly and no roots form when grown in DAS supplemented media. No differences are observed between transformed and control seeds grown in the same media without DAS.

### B. Fungal Resistance Germination Assay for Detecting Resistance to Seedling Blight

### 1. Wheat Fungal Resistance Germination Assay:

Fungal resistance germination assays in wheat are carried out substantially as described by R. H. Proctor, T. M. Hohn, and S. P. McCormick. Reduced virulence of Gibberella zeae caused by disruption of a trichothecene toxin biosynthetic gene. Mol.Plant-Microbe Interact. 8 (4):593-601, 1995.) entirety.

Inoculum consists of macroconidia of *F. gramiearum* diluted in water to 1 × 10⁶ conidia per ml. Inoculum is prepared by washing the macroconidia from V-8 juice agar cultures grown under white and near UV fluorescent lights for 7-10 days. In seedling assays, seeds of two different transgenic wheat events from Example 2 above and the wild type control are surface sterilized by washing in a 10% bleach and .05% Tween solution for approximately 15 min. and rinsed five times with sterile distilled water. The seeds are soaked in a suspension of macroconidia for approximately 10 min and then sown in vermiculite contained in 10 cm plastic pots (20 seeds per pot). Prior to sowing, the pots are filled approximately ¾ full with vermiculite and set in 2-4 cm of water until the top of the vermiculite was wet. After sowing, seeds are covered with an additional 1-2 cm of vermiculite and pots are placed individually into plastic bags and incubated in a growth chamber at 22 °C with 16 h light and 8 h dark for week. After approximately one week the pots are removed from the bags, and after two weeks, disease is evaluated by counting the number of seedlings that emerge in each pot. Controls are treated as described above except that the seeds are soaked in sterile water and 40 seeds are used.

50% and 43% of the seed from the two different transgenic plant events germinate as compared to the same transgenic seed treated with water, whereas, 17% of the wild type control germinate compared to the same seed treated with water.

### 2. Maize Fungal Resistance Germination Assay:

Inoculum is produced from *F. graminearum* cultures grown on mung bean agar (made with liquor from boiled mung beans) under 12h alternating light and dark cycles at 25°C. Spores are harvested by first flooding the plate with sterile water and then scraping the plate using a glass rod. The solution is collected and the spore concentration adjusted to 1 x 106 spores/ml with double distilled, sterile water on the day of inoculation.

Soil consisting of a sterilized mix of soil, peat, and vermiculite is inoculated with 1 ml of spore solution/liter of soil in 5 liter flats and the inoculated soil is mixed in a cement mixer for 2 minutes per load. Control treatments consist of non-infested soil. Flats are planted with 30 kernels each of the transgenic seed of the invention or wild type contol and incubated in growth chambers kept at 12.8°C (55°F) under semi-saturated soil conditions for up to 4 weeks. Light levels are under 24-hour darkness for 14 days post planting, and 12-hour light 15-24 days post planting. Labeled stakes are added to flats, flats are moved to the growth chamber and randomized.

Plant counts are initiated as soon as emergence begins, and are performed daily or every other day until there is no longer a change in plant emergence.

Symptoms of visual discoloration and damping-off of seedling emergence are determined and used to characterize the degree of plant resistance as compared to wild type controls. Plants having less visual discoloration and/or damping off of seedling emergence than the wild type control are selected.

### EXAMPLE 9: Fungal Resistance Assay

### A. Testing of transgenic wheat plants

Wheat head blight or head scab is caused by *Fusarium graminearum* (teleomorph: *Gibberella zeae). F. graminearum* cultures are grown on V-8 agar medium (made with V-8 juice) or on mung bean agar (made with liquor from boiled mung beans) under 12h alternating light and dark cycles at 25°C. Spores are harvested by first flooding the plate with sterile water and then scraping the plate using a glass rod. The solution is collected and the spore concentration adjusted to 5 x 10⁴ spores/ml with double distilled, sterile water on the day of inoculation.

Transgenic plants are obtained as described in Example 2 above, and control plants may be grown in the greenhouse until anthesis or heading. Heads are inoculated by injecting approximately 20 ul (about 1000 spores) of inoculum between the lemma and palea of one floret near the middle of each head. Some heads are left uninoculated or are inoculated with sterile water as controls. Plants are then moved to a growth chamber and incubated under high humidity for up to 21 days or plants are first incubated in a mist chamber for 72 h at 18.3 to 21.1°C (65 to 70° F) and then incubated in the greenhouse for an additional 18 days.
Transgenic plants of the invention and control plant may also be grown in the field, where heads are inoculated by spraying. Disease is evaluated by counting the number of symptomatic and asymptomatic spikelets on each inoculated head on a representative number of transgenic heads and wild type control heads and from this calculating the percentage of spikelets on each head that are symptomatic. Symptoms consist of premature whitening as compared to the control plants and in some cases necrosis of spikelets. Plants having fewer symptomatic spikelets than the wild type control are selected.
Six different transgenic plants having different copy numbers of SEQ ID NO.1 and different numbers of insertion sites according to southern data have average percent of symptomatic spikelets per head ranging from 10.40% to 31.20% compared to 44.75% for the wild type control, where the transgenic plants and controls are incubated in the green house as described above. These same transgenic plants have enzymatic activities, measured as described in example 6 above, ranging from 0.874 to 29.1 nmol/microgram/15 min.

### B. Testing of transgenic corn plants

### Corn ear rot assay.

Corn ear rot is caused by *Fusarium graminearum* (teleomorph: *Gibberella zeae). F. graminearum* cultures are grown on V-8 agar medium (made with V-8 juice) under 12h alternating light and dark cycles at 25°C. Spores are harvested by first flooding the plate with sterile water and then scraping the plate using a glass rod. The solution is collected and the spore concentration adjusted to 5 x 10⁵ spores/ml with double distilled, sterile water on the day of inoculation. Transgenic plants and control plants are grown in the greenhouse or field. Where grown in the greenhouse the transgenic and control plants are maintained in the green house until four to seven days post silk emergence when a 2 ml spore suspension is introduced into the silk channel (inside the husk cavity and above the cob). This is accomplished using an 18-gauge stainless steel hypodermic needle attached to a large syringe. In addition to silk channel inoculations, a kernel inoculation method is also used to assay disease resistance. Kernel inoculation involves the introduction of the spore suspension (approx. 0.4 ml) into a group of four kernels through multiple injections with an 18-gauge needle attached to a syringe. Disease is evaluated by visual inspection of ears harvested 5 to 7 weeks post-inoculation for visibly infected kernels. The disease rating scale for husked ears is based on a visual estimation of the percentage of visibly infected kernels on an ear as follows: 1 is 0%; 2 is 1 to 3%; 3 is 4 to 10%; 4 is 11 to 25%; 5 is 26 to 50%; 6 is 51 to 75%; 7 is 76 to 100%. Maize plants are selected that have a lower percentage of visibly infected kernels compared to the wild type control.

### EXAMPLE 10: Mycotoxin Contamination Assay

Samples are prepared for mycotoxin concentration analysis as follows. Seed is collected from transgenic plants of the invention weighed and bulked together. Where wheat seed is being assayed, wheat seed is collected from the heads of the same transgenic plants of the invention and weighed and bulked together. Where transgenic maize is being assayed, corn ears are dried to low moisture levels, ears are hand-shelled and kernels from ears of the same transgenic plant are weighed and bulked together. Each seed or kernel sample is mixed thoroughly to promote a random distribution of seed. A 50 g seed or kernel sample is ground to a fine powder in a mill (e.g. Retsch ultra centrifugal mill type ZM1, Brinkman Instruments, Inc., Rexdale, Ontario, Romer Series II Mill, Union, Missouri, USA). The concentration of the mycotoxin of interest such as, DON is then determined using the commercially available tests such as DONtest TAG^{™} mycotoxin testing system (VICAM, LP, 313 Pleasant Street, Watertown, MA 02472) or analyzed by a commercial analysis company (e.g. Romer Labs, Inc, Union, Missouri, USA or Trilogy Analytical Laboratory, Inc., Beaufort, Missouri, USA). The manufacturer's instructions are followed for all aspects of the analysis. For DONtest TAG^{™} mycotoxin testing system, a final fluorometric measurement for DON is conducted. Plants producing seed or kernels having less mycotoxin, such as DON, than the wild type control are selected.

### Example 11: Use of polynucleotide according to SEQ ID NO:1 as a selectable marker.

### A. Selectable Marker in Fungal Cells.

*Ashbya gossypi* is transformed using standard fungal transformation techniques with a DNA construct comprising a polynucleotide having the sequence of SEQ ID NO:1 operably linked to the galactosidase promoter. Transformed cells grow in media comprising DAS at a concentration ranging from 1.56ng/ml to 196 pg/ml whereas as the untransformed wild type fungal cells do not.

### B. Selectable Marker in Plant Cells.

Seed from Arabidopsis plants transformed according to Example 3 above but not yet subjected to selection is plated out in .1% agarose medium containing 0, 5, or 10 µg /ml DAS. After incubation in a growth room at 22°C with 16 hours of light and 8 hours of darkness for 2 weeks, the larger unstunted plants are transplanted from a DAS plate, and a corresponding number are transplanted from the control plate.
Leaves of Arabidopsis plants transplanted from the 5 microgram/ml plate, are assayed for enzymatic activity after a 2 week growth period, and showed 11 out of 11 unstunted plants were enzymatically active as measured by Example 6 while 9 out of 10 plants not selected by DAS were negative in the same assay. The one non-selected plant that was enzymatically active was much less active than any of the DAS selected plants assayed.
The above-disclosed embodiments are illustrative.

## Claims

1. A plant, which is resistant to a fungus that produces a trichothecene that comprises a C-3 hydroxyl group, said plant comprising a plant cell, wherein said plant cell comprises:
a) a nucleic acid sequence, the complement of which hybridises to SEQ ID NO: 1 in 7% sodium dodecyl sulphate, 0.5M NaPO₄, 1mM EDTA at 50°C with washing in 0.5X SSC, 0.1 % SDS at 50°C;
b) a nucleic acid sequence having at least 90% sequence identity to SEQ ID NO: 1; or
c) a nucleic acid sequence which encodes the amino acid sequence of SEQ ID NO: 2 said nucleic acid sequence encoding a protein which has trichothecene 3-*O*-acetyltransferase activity.

2. The plant of claim 1, wherein the plant cell comprises a nucleic acid sequence, the complement of which hybridises to SEQ ID NO: 1 in 7% sodium dodecyl sulphate, 0.5M NaPO₄, 1mM EDTA at 50°C with washing in 0.1X SSC, 0.1 % SDS at 50°C.

3. The plant of claim 1 or claim 2, wherein the plant is resistant to *Fusarium* infection.

4. The plant of any one of claims 1 to 3, which is a monocot.

5. The plant of claim 4 which is a corn plant.

6. A seed of the plant according to any one of claims 1 to 5, wherein said seed comprises the nucleic acid sequence of SEQ ID NO: 1.

7. A method for producing a wheat plant that is resistant to a fungus that produces a trichothecene that comprises a C-3 hydroxyl group comprising the steps of
a) transforming a wheat plant cell with a heterologous gene comprising a nucleic acid sequence as defined in claim 1;
b) expressing the gene product of said gene at a biologically significant level;
c) regenerating the plant cell into a plant; and
d) selecting a plant with increased resistance to the fungus; and, optionally,
e) selfing or outcrossing the plant obtained in step d).

8. The method of claim 7 wherein the fungus is of the genus *Fusarium.*

9. A method of preventing mycotoxin contamination of a plant and/or a plant seed, comprising growing a plant of any one of claims 1 to 5 or a plant produced by a method according to claim 7 or claim 8, preferably in an area with moderate to severe fungal infestation, wherein the fungus is of the genus *Fusarium* and wherein the mycotoxin is a trichothecene comprising a C-3 hydroxyl group.

10. A method for reducing and/or preventing the growth of a fungus of the genus *Fusarium* on a plant, said fungus being capable of producing a trichothecene comprising a C-3 hydroxyl group, said method comprising growing the plant of any one of claims 1 to 5 or a plant produced by a method according to claim 7 or claim 8, preferably in an area with moderate to severe fungal infestation.

11. A method of producing seed wherein the plant grown from the seed is fungal resistant, comprising selfing or outcrossing the plant of any one of claims 1 to 5 or a plant produced by a method according to claim 7 or claim 8.

12. The method of claim 11, wherein the seed is resistant to fungi of the genus *Fusarium.*

## Patentansprüche

1. Pflanze, die resistent gegen einen Pilz ist, der ein Trichothecen erzeugt, das eine C-3-Hydroxyl-Gruppe umfaßt, wobei die Pflanze eine Pflanzenzelle umfaßt, wobei die Pflanzenzelle folgendes umfaßt:
a) eine Nukleinsäuresequenz, deren Komplement in 7 % Natriumdodecylsulfat, 0,5 M NaPO₄, 1 mM EDTA bei 50°C mit Waschen in 0,5 X SSC, 0,1 % SDS bei 50°C an SEQ ID NO:1 hybridisiert;
b) eine Nukleinsäure mit wenigstens 90 % Sequenzidentität zu SEQ ID NO:1; oder
c) eine Nukleinsäuresequenz, die die Aminosäuresequenz gemäss SEQ ID NO:2 codiert, wobei die Nukleinsäuresequenz für ein Protein codiert, das Trichothecen-3-O-acetyltransferase-Aktivität aufweist.

2. Pflanze gemäß Anspruch 1, wobei die Pflanzenzelle eine Nukleinsäuresequenz umfaßt, deren Komplement in 7 % Natriumdodecylsulfat, 0,5 M NaPO4, 1 mM EDTA bei 50°C mit Waschen in 0,1 X SSC, 0,1 % SDS bei 50°C an SEQ ID NO:1 hybridisiert.

3. Pflanze gemäß Anspruch 1 oder Anspruch 2, wobei die Pflanze resistent gegen eine *Fusarium*-Infektion ist.

4. Pflanze gemäß einem der Ansprüche 1 bis 3, die eine einkeimblättrige Pflanze ist.

5. Pflanze gemäß Anspruch 4, die eine Getreidepflanze ist.

6. Saatgut der Pflanze gemäß einem der Ansprüche 1 bis 5, wobei das Saatgut die Nukleinsäuresequenz gemäss SEQ ID NO:1 umfaßt.

7. Verfahren zur Erzeugung einer Weizenpflanze, die resistent gegen einen Pilz ist, der ein Trichothecen erzeugt, das eine C-3-Hydroxyl-Gruppe umfaßt, wobei das Verfahren die folgenden Schritte umfasst:
a) Transformieren einer Weizenpflanzenzelle mit einem heterologen Gen, das die Nukleinsäuresequenz gemäß Anspruch 1 umfaßt;
b) Exprimieren des Genprodukts dieses Gens auf einem biologisch bedeutsamen Niveau;
c) Regenerieren der Pflanzenzelle zu einer Pflanze; und
d) Selektieren einer Pflanze mit erhöhter Resistenz gegen den Pilz; und gegebenenfalls
e) Selbsten oder Auskreuzen der in Schritt d) erhaltenen Pflanze.

8. Verfahren gemäß Anspruch 7, wobei der Pilz aus der Gattung *Fusarium* ist.

9. Verfahren zur Prävention einer Mycotoxin-Kontamination einer Pflanze und/oder eines Pflanzensaatguts, welches das Kultivieren einer Pflanze gemäß einem der Ansprüche 1 bis 5 oder einer durch ein Verfahren gemäß Anspruch 7 oder 8 erzeugten Pflanze umfasst, bevorzugt in einem Gebiet mit moderatem bis schwerem Pilzbefall, wobei der Pilz aus der Gattung *Fusarium* ist und wobei das Mycotoxin ein Trichotecen ist, das eine C-3-Hydroxyl-Gruppe umfaßt.

10. Verfahren zur Verringerung und/oder Prävention des Wachstums eines Pilzes der Gattung *Fusarium* auf einer Pflanze, wobei der Pilz ein Trichothecen erzeugen kann, das eine C-3-Hydroxyl-Gruppe umfaßt, wobei das Verfahren das Kultivieren der Pflanze gemäß einem der Ansprüche 1 bis 5 oder einer durch ein Verfahren gemäß Anspruch 7 oder 8 erzeugten Pflanze umfaßt, bevorzugt in einem Gebiet mit moderatem bis schwerem Pilzbefall.

11. Verfahren zur Erzeugung von Saatgut, wobei die aus dem Saatgut kultivierte Pflanze pilzresistent ist, wobei das Verfahren das Selbsten oder Auskreuzen der Pflanze gemäß einem der Ansprüche 1 bis 5 oder einer durch ein Verfahren gemäß Anspruch 7 oder 8 erzeugten Pflanze umfasst.

12. Verfahren gemäß Anspruch 11, wobei das Saatgut gegen Pilze der Gattung Fusarium resistent ist.

## Revendications

1. Plante, qui est résistante à un champignon qui produit un trichothécène qui comprend un groupe hydroxyle en C-3, ladite plante comprenant une cellule végétale, ladite cellule végétale comprenant:
a) une séquence d'acide nucléique dont le complément s'hybride à SEQ ID NO: 1 dans 7 % de dodécylsulfate de sodium, NaPO₄ 0,5 M, EDTA 1 mM à 50°C avec un lavage dans 0,5 X SSC, 0,1 % de SDS à 50°C;
b) une séquence d'acide nucléique ayant une identité de séquence d'au moins 90 % avec SEQ ID NO: 1; ou
c) une séquence d'acide nucléique qui code pour la séquence d'aminoacides de SEQ ID NO: 2,
ladite séquence d'acide nucléique codant pour une protéine qui a une activité de trichothécène-3-O-acétyltransférase.

2. Plante selon la revendication 1, dans laquelle la cellule végétale comprend une séquence d'acide nucléique dont le complément s'hybride à SEQ ID NO: 1 dans 7 % de dodécylsulfate de sodium, NaPO₄ 0,5 M, EDTA 1 mM à 50°C avec un lavage dans 0,1 X SSC, 0,1 % de SDS à 50°C.

3. Plante selon la revendication 1 ou la revendication 2, la plante étant résistante à l'infection par *Fusarium.*

4. Plante selon l'une quelconque des revendications 1 à 3, qui est une monocotylédone.

5. Plante selon la revendication 4, qui est une plante de maïs.

6. Graine de la plante selon l'une quelconque des revendications 1 à 5, ladite graine comprenant la séquence d'acide nucléique de SEQ ID N° 1.

7. Procédé de production d'une plante de blé qui est résistante à un champignon qui produit un trichothécène qui comprend un groupe hydroxyle en C-3, comprenant les étapes de:
a) transformation d'une cellule végétale de blé avec un gène hétérologue comprenant une séquence d'acide nucléique telle que définie dans la revendication 1;
b) expression du produit génétique dudit gène à un niveau biologiquement significatif;
c) régénération de la cellule végétale en une plante; et
d) sélection d'une plante ayant une résistance accrue au champignon; et, éventuellement,
e) autofécondation ou croisement éloigné de la plante obtenue dans l'étape d).

8. Procédé selon la revendication 7, dans lequel le champignon est du genre *Fusarium.*

9. Procédé de prévention de la contamination par une mycotoxine d'une plante et/ou d'une graine de plante, comprenant la culture d'une plante selon l'une quelconque des revendications 1 à 5 ou d'une plante produite par un procédé selon la revendication 7 ou la revendication 8, de préférence dans une zone d'infestation fongique moyenne à importante, où le champignon est du genre *Fusarium* et la mycotoxine est un trichothécène comprenant un groupe hydroxyle en C-3.

10. Procédé de réduction et/ou de prévention de la croissance d'un champignon du genre *Fusarium* sur une plante, ledit champignon étant capable de produire un trichothécène comprenant un groupe hydroxyle en C-3, ledit procédé comprenant la culture de la plante selon l'une quelconque des revendications 1 à 5 ou d'une plante produite par un procédé selon la revendication 7 ou la revendication 8, de préférence dans une zone d'infestation fongique moyenne à importante.

11. Procédé de production d'une graine, la plante cultivée à partir de la graine étant résistante à des champignons, comprenant l'autofécondation ou le croisement éloigné de la plante selon l'une quelconque des revendications 1 à 5 ou d'une plante produite par un procédé selon la revendication 7 ou la revendication 8.

12. Procédé selon la revendication 11, dans lequel la graine est résistante aux champignons du genre *Fusarium.*


### SEQUENCE LISTING

<110> Hohn, T.
   Salmeron, J.
   Peters, C.
   Kendra, D.
   Reinders, J.
   Kuznia, R.
   Dill-Mackey, R.
<120> Transgenic Plant and Methods
<130> sequencelist
<140>
   <141>
<160> 11
<170> PatentIn Ver. 2.0
<210> 1
   <211> 1403
   <212> DNA
   <213> Fusarium sporotrichioides
<400> 1
   atcaaaatgg ccgcaacaag cagcacaagc agccagtctt ttgacataga gctcgacatc 60
   atcggccagc aaccgcctct tctttcaatc tacacccaga tcagtctcgt ttaccccgtc 120
   tctgatccct cccagtatcc caccatcgtc agcacccttg aggaaggcct aaaacgcctc 180
   tctcaaacct tcccatgggt cgcgggccag gtcaagaccg agggcatcag cgaaggaaac 240
   acaggaactt ccaagatcat tccatatgag gagacacccc gtcttgtggt gaaagacctc 300
   cgtgatgatt cctcagcgcc aacgatcgag gggttgagaa aggcgggttt ccccttagag 360
   atgtttgacg agaacgtcgt cgctccgagg aagacattag ctatcggacc tggcaatggc 420
   cccaacgacc cgaagcctgt gttgctattg cagctcaact tcattaaggg cggactcatt 480
   ctcaccgtca acggacaaca tggtgctatg gacatgacag gacaagatgc aattattcgt 540
   cttctctcca aggcgtgccg caacgaatca ttcaccgagg aggaaatctc ggccatgaac 600
   ctcgatcgca agacggtagt ccctctcctt gaaaactaca aagttggtcc tgagctagac 660
   caccagatcg ccaaacctgc gcctgctggc gacgctccac ccgcaccggc caaggcaagc 720
   tgggcgttct tttcattcac tcccaaggcc ctctcggagc tgaaagacgc agccacaaag 780
   actcttgacg cgtcgtccaa gtttgtgtca actgatgatg ctctttcggc gtttatctgg 840
   caatcaacct cgcgcgtacg tctcgcaaga ttggatgctt ccacacctac tgaattctgc 900
   cgcgctgtcg acatgcgggg cccaatgggc gtatcaagca catacccagg ccttcttcaa 960
   aacatgacct accatgactc gaccgtcgcc gaaatcgcca acgaaccact tggcgcaaca 1020
   gcatcacgcc tgcgctcgga actcaacagt gatcgtttgc gcagacgaac acaagctttg 1080
   gcgacgtaca tgcatggcct gcctgacaag tcgagcgtct ccctgaccgc cgatgcgaat 1140
   ccgtcaagca gcatcatgct gagttcctgg gccaaggtgg gatgctggga gtatgacttt 1200
   gggtttggac tgggtaagcc tgagagtgtg agaagacctc gctttgaacc ttttgagagt 1260
   ttgatgtact ttatgcccaa gaagcctgat ggggagttta cggcgtccat ttctctgagg 1320
   gatgaggata tggagagact aaaggcggat gaggagtgga caaagtacgc aaagtatatt 1380
   gggtagatag tttactagac tac                                          1403
<210> 2
   <211> 459
   <212> PRT
   <213> Fusarium sporotrichioides
<400> 2
   Met Ala Ala Thr Ser Ser Thr Ser Ser Gln Ser Phe Asp Ile Glu Leu
     1               5                  10                  15
   Asp Ile Ile Gly Gln Gln Pro Pro Leu Leu Ser Ile Tyr Thr Gln Ile
               20                  25                  30
   Ser Leu Val Tyr Pro Val Ser Asp Pro Ser Gln Tyr Pro Thr Ile Val
           35                  40                  45
   Ser Thr Leu Glu Glu Gly Leu Lys Arg Leu Ser Gln Thr Phe Pro Trp
       50                  55                  60
   Val Ala Gly Gln Val Lys Thr Glu Gly Ile Ser Glu Gly Asn Thr Gly
   65                  70                  75                  80
   Thr Ser Lys Ile Ile Pro Tyr Glu Glu Thr Pro Arg Leu Val Val Lys
                   85                  90                  95
   Asp Leu Arg Asp Asp Ser Ser Ala Pro Thr Ile Glu Gly Leu Arg Lys
               100                 105                 110
   Ala Gly Phe Pro Leu Glu Met Phe Asp Glu Asn Val Val Ala Pro Arg
           115                 120                 125
   Lys Thr Leu Ala Ile Gly Pro Gly Asn Gly Pro Asn Asp Pro Lys Pro
       130                 135                 140
   Val Leu Leu Leu Gln Leu Asn Phe Ile Lys Gly Gly Leu Ile Leu Thr
   145                 150                 155                 160
   Val Asn Gly Gln His Gly Ala Met Asp Met Thr Gly Gln Asp Ala Ile
                   165                 170                 175
   Ile Arg Leu Leu Ser Lys Ala Cys Arg Asn Glu Ser Phe Thr Glu Glu
               180                 185                 190
   Glu Ile Ser Ala Met Asn Leu Asp Arg Lys Thr Val Val Pro Leu Leu
           195                 200                 205
   Glu Asn Tyr Lys Val Gly Pro Glu Leu Asp His Gln Ile Ala Lys Pro
       210                 215                 220
   Ala Pro Ala Gly Asp Ala Pro Pro Ala Pro Ala Lys Ala Ser Trp Ala
   225                 230                 235                 240
   Phe Phe Ser Phe Thr Pro Lys Ala Leu Ser Glu Leu Lys Asp Ala Ala
                   245                 250                 255
   Thr Lys Thr Leu Asp Ala Ser Ser Lys Phe Val Ser Thr Asp Asp Ala
               260                 265                 270
   Leu Ser Ala Phe Ile Trp Gln Ser Thr Ser Arg Val Arg Leu Ala Arg
           275                 280                 285
   Leu Asp Ala Ser Thr Pro Thr Glu Phe Cys Arg Ala Val Asp Met Arg
       290                 295                 300
   Gly Pro Met Gly Val Ser Ser Thr Tyr Pro Gly Leu Leu Gln Asn Met
   305                 310                 315                 320
   Thr Tyr His Asp Ser Thr Val Ala Glu Ile Ala Asn Glu Pro Leu Gly
                   325                 330                 335
   Ala Thr Ala Ser Arg Leu Arg Ser Glu Leu Asn Ser Asp Arg Leu Arg
   340 345 350
   Arg Arg Thr Gln Ala Leu Ala Thr Tyr Met His Gly Leu Pro Asp Lys
   355 360 365
   Ser Ser Val Ser Leu Thr Ala Asp Ala Asn Pro Ser Ser Ser Ile Met
   370 375 380
   Leu Ser Ser Trp Ala Lys Val Gly Cys Trp Glu Tyr Asp Phe Gly Phe
   385 390 395 400
   Gly Leu Gly Lys Pro Glu Ser Val Arg Arg Pro Arg Phe Glu Pro Phe
   405 410 415
   Glu Ser Leu Met Tyr Phe Met Pro Lys Lys Pro Asp Gly Glu Phe Thr
   420 425 430
   Ala Ser Ile Ser Leu Arg Asp Glu Asp Met Glu Arg Leu Lys Ala Asp
   435 440 445
   Glu Glu Trp Thr Lys Tyr Ala Lys Tyr Ile Gly
   450 455
<210> 3
   <211> 20
   <212> DNA
   <213> Artificial Sequence
<220>
   <223> Description of Artificial Sequence: DNA Primer
<400> 3
   acgaatcatt caccgaggag 20
<210> 4
   <211> 20
   <212> DNA
   <213> Artificial Sequence
<220>
   <223> Description of Artificial Sequence: DNA Primer
<400> 4
   ctcacactct caggcttacc 20
<210> 5
   <211> 1356
   <212> DNA
   <213> Fusarium graminearum
<400> 5
   atggctttca agatacagct cgacaccctc ggccagctac caggcctcct ttcgatctac 60
   acccaaatca gtctcctcta ccccgtctct gattcctctc aatatcccac tattgtcagc 120
   accttcgagc aaggtcttaa gcgcttctcc gaagccgtcc catgggtcgc aggccaggtc 180
   aaagccgagg gcattagcga gggaaacaca ggaacttcct ttatcgtccc ttttgaggac 240
   gttcctcgtg ttgtagtgaa agacctccgc gatgatcctt cagcgcccac gatcgagggt 300
   atgagaaagg cgggataccc tatggcgatg tttgacgaga acatcatcgc gccaaggaag 360
   acgttaccta ttggacctgg tactggtccc gacgacccaa agcctgtaat tctattgcag 420
   ctcaacttca tcaagggcgg actcatcctc actgtcaacg gacagcacgg tgctatggat 480
   atggtaggcc aagatgcggt gatccgtcta ctctccaagg cgtgccgtaa cgacccattc 540
   accgaagagg aaatgacggc catgaacctc gatcgcaaga cgatagttcc ttaccttgaa 600
   aactatacga ttggccccga ggtagatcat cagattgtca aagctgatgt agctggtggt 660
   gacgctgttc tcacgccggt cagtgcaagc tgggcgttct tcacattcag ccccaaggcc 720
   atgtcagagc tcaaggatgc tgctaccaag actcttgacg catcaacaaa gttcgtgtcg 780
   actgacgatg ctctttcggc gttcatctgg aaatcggcct ctcgcgtgcg tctcgaaaga 840
   atcgatggct ctgcacctac cgagttctgc cgtgctgttg atgctcgacc ggcaatgggt 900
   gtctcgaaca actacccagg ccttcttcaa aacatgacct accacaactc gaccatcggc 960
   gaaatcgcca acgagtcact cggcgcaaca gcatcacgcc ttcgttcaga actcgacccc 1020
   gcgagcatgc gccagcgaac aagaggtctc gcgacgtacc tgcacaacaa ccccgacaag 1080
   tccaacgtat ccctgacggc tgatgcggac ccatctacca gcgtcatgct gagttcttgg 1140
   gccaaggtgg gactctggga ttacgacttt gggctcggac tgggtaagcc cgagactgtg 1200
   agacggccaa tctttgagcc tgttgagagc ttgatgtact ttatgcccaa gaagcctgat 1260
   ggcgagttct gtgcggcgct ttctctgagg gatgaggata tggaccgatt gaaggcggat 1320
   aaggagtgga ccaagtatgc gcagtacgtt ggttag                           1356
<210> 6
   <211> 451
   <212> PRT
   <213> Fusarium graminearum
<400> 6
   Met Ala Phe Lys Ile Gln Leu Asp Thr Leu Gly Gln Leu Pro Gly Leu
   1               5                   10                  15
   Leu Ser Ile Tyr Thr Gln Ile Ser Leu Leu Tyr Pro Val Ser Asp Ser
               20                  25                  30
   Ser Gln Tyr Pro Thr Ile Val Ser Thr Phe Glu Gln Gly Leu Lys Arg
           35                  40                  45
   Phe Ser Glu Ala Val Pro Trp Val Ala Gly Gln Val Lys Ala Glu Gly
       50                  55                  60
   Ile Ser Glu Gly Asn Thr Gly Thr Ser Phe Ile Val Pro Phe Glu Asp
   65                  70                  75                  80
   Val Pro Arg Val Val Val Lys Asp Leu Arg Asp Asp Pro Ser Ala Pro
                   85                  90                  95
   Thr Ile Glu Gly Met Arg Lys Ala Gly Tyr Pro Met Ala Met Phe Asp
               100                 105                 110
   Glu Asn Ile Ile Ala Pro Arg Lys Thr Leu Pro Ile Gly Pro Gly Thr
           115                 120                 125
   Gly Pro Asp Asp Pro Lys Pro Val Ile Leu Leu Gln Leu Asn Phe Ile
       130                 135                 140
   Lys Gly Gly Leu Ile Leu Thr Val Asn Gly Gln His Gly Ala Met Asp
   145                 150                 155                 160
   Met Val Gly Gln Asp Ala Val Ile Arg Leu Leu Ser Lys Ala Cys Arg
                   165                 170                 175
   Asn Asp Pro Phe Thr Glu Glu Glu Met Thr Ala Met Asn Leu Asp Arg
               180                 185                 190
   Lys Thr Ile Val Pro Tyr Leu Glu Asn Tyr Thr Ile Gly Pro Glu Val
           195                 200                 205
   Asp His Gln Ile Val Lys Ala Asp Val Ala Gly Gly Asp Ala Val Leu
           210                     215                     220
   Thr Pro Val Ser Ala Ser Trp Ala Phe Phe Thr Phe Ser Pro Lys Ala
   225                 230                 235                 240
   Met Ser Glu Leu Lys Asp Ala Ala Thr Lys Thr Leu Asp Ala Ser Thr
                   245                 250                 255
   Lys Phe Val Ser Thr Asp Asp Ala Leu Ser Ala Phe Ile Trp Lys Ser
               260                 265                 270
   Ala Ser Arg Val Arg Leu Glu Arg Ile Asp Gly Ser Ala Pro Thr Glu
           275                 280                 285
   Phe Cys Arg Ala Val Asp Ala Arg Pro Ala Met Gly Val Ser Asn Asn
       290                 295                 300
   Tyr Pro Gly Leu Leu Gln Asn Met Thr Tyr His Asn Ser Thr Ile Gly
   305                 310                 315                 320
   Glu Ile Ala Asn Glu Ser Leu Gly Ala Thr Ala Ser Arg Leu Arg Ser
                   325                 330                 335
   Glu Leu Asp Pro Ala Ser Met Arg Gln Arg Thr Arg Gly Leu Ala Thr
               340                 345                 350
   Tyr Leu His Asn Asn Pro Asp Lys Ser Asn Val Ser Leu Thr Ala Asp
           355                 360                 365
   Ala Asp Pro Ser Thr Ser Val Met Leu Ser Ser Trp Ala Lys Val Gly
       370                 375                 380
   Leu Trp Asp Tyr Asp Phe Gly Leu Gly Leu Gly Lys Pro Glu Thr Val
   385                 390                 395                 400
   Arg Arg Pro Ile Phe Glu Pro Val Glu Ser Leu Met Tyr Phe Met Pro
                   405                 410                 415
   Lys Lys Pro Asp Gly Glu Phe Cys Ala Ala Leu Ser Leu Arg Asp Glu
               420                 425                 430
   Asp Met Asp Arg Leu Lys Ala Asp Lys Glu Trp Thr Lys Tyr Ala Gln
           435                 440                 445
   Tyr Val Gly
       450
<210> 7
   <211> 1425
   <212> DNA
   <213> Saccharomyces cerevisiae
<400> 7
   atgtttagag tcaagatcat ctctcagaaa cgtacaaaaa gtgtacagat gctagaaaac 60
   gatcaacttg atattttggg acaacaacct tcgctataca aactatacac tcaaatatgc 120
   tctatctacc gtgtaccaga tccttctgct catgaccata tcgtaaatac cttaacaaga 180
   ggacttgaaa cattggctaa aaatttccag tggctagcag gaaatgtcgt aaatgaaggt 240
   gctgacgaag gtaacactgg tacctacaga attgtcccgt cagacaaaat tccacttatc 300
   gtccaagatc ttcgagaaga tctgtctgcc ccaacaatgg attcgcttga aaaagctgac 360
   tttcctatct acatgttaga cgaaaagact tttgcgcctt gcatgactat caatccacct 420
   ggaaacacta taggtatggc cgccaagagt gggcctgtat ttgcagttca agcaaacttt 480
   atctccggcg gcctcgtctt aactattgtc gggcagcaca atattatgga tataacagga 540
   caggaaagta tcatcaactt gctcaataaa tcttgccacc aaaaaccttt ctctgatgaa 600
   gaactgctca ttggaaatat agataaaagc aaatctattc ctttgtttga tgaaacttgg 660
   gaacccgaca ccacgctagt tcatgaaata gtggaaacct ctagaaatac aagtggagag 720
   gaaaaggaac agtcttgttc ttcgaactct acttgggctt atgttgaatt ttctgctatc 780
   tcattgcaga atctgaggat tttggcaatg cagacatgta cttctggcac aaaatttgtc 840
   tccactgatg atatcgtcac tgctttcatc tggaaatcag tttctcgagc ccgtttatct 900
   cgacttaaac cagaaacgaa atcaaattta gggcgtgctg tggatgttag aaaacggcta 960
   ggactccccg aaacgtatcc agggttatta gtcaacatga cctttaatac aggttccctg 1020
   aaaagcttgg atcataaaag tttgggcgtt cttgcatcac agattcgcag gaagctagac 1080
   cctaaagtct tcgatttggc ctataataca tgcgcacttg ctacgctcct tagccgatgc 1140
   ccggacaaga ctaaggtttc tatacctcaa ccaattgata ctttatctgg aattatggtc 1200
   agttcgtggg caaaagtcag cctgtatgac gttgatttca atctagggct tgggaagccc 1260
   aagagtgtac gacggccgcg cttcatttcc cttgagagcc taatatattt tatgcctaga 1320
   tcctccagag gtgaaatggt ggttgctctt tgccttagag ataaagattg ggagtgcctg 1380
   aatgcggata aagaatggac aaattatgct acacatatag gatga                1425
<210> 8
   <211> 474
   <212> PRT
   <213> Saccharomyces cerevisiae
<400> 8
   Met Phe Arg Val Lys Ile Ile Ser Gln Lys Arg Thr Lys Ser Val Gln
   1 5 10 15
   Met Leu Glu Asn Asp Gln Leu Asp Ile Leu Gly Gln Gln Pro Ser Leu
   20 25 30
   Tyr Lys Leu Tyr Thr Gln Ile Cys Ser Ile Tyr Arg Val Pro Asp Pro
   35 40 45
   Ser Ala His Asp His Ile Val Asn Thr Leu Thr Arg Gly Leu Glu Thr
   50 55 60
   Leu Ala Lys Asn Phe Gln Trp Leu Ala Gly Asn Val Val Asn Glu Gly
   65 70 75 80
   Ala Asp Glu Gly Asn Thr Gly Thr Tyr Arg Ile Val Pro Ser Asp Lys
   85 90 95
   Ile Pro Leu Ile Val Gln Asp Leu Arg Glu Asp Leu Ser Ala Pro Thr
   100 105 110
   Met Asp Ser Leu Glu Lys Ala Asp Phe Pro Ile Tyr Met Leu Asp Glu
   115 120 125
   Lys Thr Phe Ala Pro Cys Met Thr Ile Asn Pro Pro Gly Asn Thr Ile
   130 135 140
   Gly Met Ala Ala Lys Ser Gly Pro Val Phe Ala Val Gln Ala Asn Phe
   145 150 155 160
   Ile Ser Gly Gly Leu Val Leu Thr Ile Val Gly Gln His Asn Ile Met
   165 170 175
   Asp Ile Thr Gly Gln Glu Ser Ile Ile Asn Leu Leu Asn Lys Ser Cys
   180 185 190
   His Gln Lys Pro Phe Ser Asp Glu Glu Leu Leu Ile Gly Asn Ile Asp
                   195                     200                     205
   Lys Ser Lys Ser Ile Pro Leu Phe Asp Glu Thr Trp Glu Pro Asp Thr
       210                 215                 220
   Thr Leu Val His Glu Ile Val Glu Thr Ser Arg Asn Thr Ser Gly Glu
   225                 230                 235                 240
   Glu Lys Glu Gln Ser Cys Ser Ser Asn Ser Thr Trp Ala Tyr Val Glu
                   245                 250                 255
   Phe Ser Ala Ile Ser Leu Gln Asn Leu Arg Ile Leu Ala Met Gln Thr
               260                 265                 270
   Cys Thr Ser Gly Thr Lys Phe Val Ser Thr Asp Asp Ile Val Thr Ala
           275                 280                 285
   Phe Ile Trp Lys Ser Val Ser Arg Ala Arg Leu Ser Arg Leu Lys Pro
       290                 295                 300
   Glu Thr Lys Ser Asn Leu Gly Arg Ala Val Asp Val Arg Lys Arg Leu
   305                 310                 315                 320
   Gly Leu Pro Glu Thr Tyr Pro Gly Leu Leu Val Asn Met Thr Phe Asn
                   325                 330                 335
   Thr Gly Ser Leu Lys Ser Leu Asp His Lys Ser Leu Gly Val Leu Ala
               340                 345                 350
   Ser Gln Ile Arg Arg Lys Leu Asp Pro Lys Val Phe Asp Leu Ala Tyr
           355                 360                 365
   Asn Thr Cys Ala Leu Ala Thr Leu Leu Ser Arg Cys Pro Asp Lys Thr
       370                 375                 380
   Lys Val Ser Ile Pro Gln Pro Ile Asp Thr Leu Ser Gly Ile Met Val
   385                 390                 395                 400
   Ser Ser Trp Ala Lys Val Ser Leu Tyr Asp Val Asp Phe Asn Leu Gly
                   405                 410                 415
   Leu Gly Lys Pro Lys Ser Val Arg Arg Pro Arg Phe Ile Ser Leu Glu
               420                 425                 430
   Ser Leu Ile Tyr Phe Met Pro Arg Ser Ser Arg Gly Glu Met Val Val
           435                 440                 445
   Ala Leu Cys Leu Arg Asp Lys Asp Trp Glu Cys Leu Asn Ala Asp Lys
       450                 455                 460
   Glu Trp Thr Asn Tyr Ala Thr His Ile Gly
   465                 470
<210> 9
   <211> 6111
   <212> DNA
   <213> Plasmid
<400> 9
   aagcttgcat gcctgcagtg cagcgtgacc cggtcgtgcc cctctctaga gataatgagc 60
   attgcatgtc taagttataa aaaattacca catatttttt ttgtcacact tgtttgaagt 120
   gcagtttatc tatctttata catatattta aactttactc tacgaataat ataatctata 180
   gtactacaat aatatcagtg ttttagagaa tcatataaat gaacagttag acatggtcta 240
   aaggacaatt gagtattttg acaacaggac tctacagttt tatcttttta gtgtgcatgt 300
   gttctccttt ttttttgcaa atagcttcac ctatataata cttcatccat tttattagta 360
   catccattta gggtttaggg ttaatggttt ttatagacta atttttttag tacatctatt 420
   ttattctatt ttagcctcta aattaagaaa actaaaactc tattttagtt tttttattta 480
   ataatttaga tataaaatag aataaaataa agtgactaaa aattaaacaa atacccttta 540
   agaaattaaa aaaactaagg aaacattttt cttgtttcga gtagataatg ccagcctgtt 600
   aaacgccgtc gacgagtcta acggacacca accagcgaac cagcagcgtc gcgtcgggcc 660
   aagcgaagca gacggcacgg catctctgtc gctgcctctg gacccctctc gagagttccg 720
   ctccaccgtt ggacttgctc cgctgtcggc atccagaaat tgcgtggcgg agcggcagac 780
   gtgagccggc acggcaggcg gcctcctcct cctctcacgg caccggcagc tacgggggat 840
   tcctttccca ccgctccttc gctttccctt cctcgcccgc cgtaataaat agacaccccc 900
   tccacaccct ctttccccaa cctcgtgttg ttcggagcgc acacacacac aaccagatct 960
   cccccaaatc cacccgtcgg cacctccgct tcaaggtacg ccgctcgtcc tccccccccc 1020
   cccctctcta ccttctctag atcggcgttc cggtccatgg ttagggcccg gtagttctac 1080
   ttctgttcat gtttgtgtta gatccgtgtt tgtgttagat ccgtgctgct agcgttcgta 1140
   cacggatgcg acctgtacgt cagacacgtt ctgattgcta acttgccagt gtttctcttt 1200
   ggggaatcct gggatggctc tagccgttcc gcagacggga tcgatttcat gatttttttt 1260
   gtttcgttgc atagggtttg gtttgccctt ttcctttatt tcaatatatg ccgtgcactt 1320
   gtttgtcggg tcatcttttc atgctttttt ttgtcttggt tgtgatgatg tggtctggtt 1380
   gggcggtcgt tctagatcgg agtagaattc tgtttcaaac tacctggtgg atttattaat 1440
   tttggatctg tatgtgtgtg ccatacatat tcatagttac gaattgaaga tgatggatgg 1500
   aaatatcgat ctaggatagg tatacatgtt gatgcgggtt ttactgatgc atatacagag 1560
   atgctttttg ttcgcttggt tgtgatgatg tggtgtggtt gggcggtcgt tcattcgttc 1620
   tagatcggag tagaatactg tttcaaacta cctggtgtat ttattaattt tggaactgta 1680
   tgtgtgtgtc atacatcttc atagttacga gtttaagatg gatggaaata tcgatctagg 1740
   ataggtatac atgttgatgt gggttttact gatgcatata catgatggca tatgcagcat 1800
   ctattcatat gctctaacct tgagtaccta tctattataa taaacaagta tgttttataa 1860
   ttattttgat cttgatatac ttggatgatg gcatatgcag cagctatatg tggatttttt 1920
   tagccctgcc ttcatacgct atttatttgc ttggtactgt ttcttttgtc gatgctcacc 1980
   ctgttgtttg gtgttacttc tgcagggatc cccgatcatg caaaaactca ttaactcagt 2040
   gcaaaactat gcctggggca gcaaaacggc gttgactgaa ctttatggta tggaaaatcc 2100
   gtccagccag ccgatggccg agctgtggat gggcgcacat ccgaaaagca gttcacgagt 2160
   gcagaatgcc gccggagata tcgtttcact gcgtgatgtg attgagagtg ataaatcgac 2220
   tctgctcgga gaggccgttg ccaaacgctt tggcgaactg cctttcctgt tcaaagtatt 2280
   atgcgcagca cagccactct ccattcaggt tcatccaaac aaacacaatt ctgaaatcgg 2340
   ttttgccaaa gaaaatgccg caggtatccc gatggatgcc gccgagcgta actataaaga 2400
   tcctaaccac aagccggagc tggtttttgc gctgacgcct ttccttgcga tgaacgcgtt 2460
   tcgtgaattt tccgagattg tctccctact ccagccggtc gcaggtgcac atccggcgat 2520
   tgctcacttt ttacaacagc ctgatgccga acgtttaagc gaactgttcg ccagcctgtt 2580
   gaatatgcag ggtgaagaaa aatcccgcgc gctggcgatt ttaaaatcgg ccctcgatag 2640
   ccagcagggt gaaccgtggc aaacgattcg tttaatttct gaattttacc cggaagacag 2700
   cggtctgttc tccccgctat tgctgaatgt ggtgaaattg aaccctggcg aagcgatgtt 2760
   cctgttcgct gaaacaccgc acgcttacct gcaaggcgtg gcgctggaag tgatggcaaa 2820
   ctccgataac gtgctgcgtg cgggtctgac gcctaaatac attgatattc cggaactggt 2880
   tgccaatgtg aaattcgaag ccaaaccggc taaccagttg ttgacccagc cggtgaaaca 2940
   aggtgcagaa ctggacttcc cgattccagt ggatgatttt gccttctcgc tgcatgacct 3000
   tagtgataaa gaaaccacca ttagccagca gagtgccgcc attttgttct gcgtcgaagg 3060
   cgatgcaacg ttgtggaaag gttctcagca gttacagctt aaaccgggtg aatcagcgtt 3120
   tattgccgcc aacgaatcac cggtgactgt caaaggccac ggccgtttag cgcgtgttta 3180
   caacaagctg taagagctta ctgaaaaaat taacatctct tgctaagctg ggagctctag 3240
   atctgttctg cacaaagtgg agtagtcagt catcgatcag gaaccagaca ccagactttt 3300
   attcatacag tgaagtgaag tgaagtgcag tgcagtgagt tgctggtttt tgtacaactt 3360
   agtatgtatt tgtatttgta aaatacttct atcaataaaa tttctaattc ctaaaaccaa 3420
   aatccagtgg gtaccgaatt cactggccgt cgttttacaa cgtcgtgact gggaaaaccc 3480
   tggcgttacc caacttaatc gccttgcagc acatccccct ttcgccagct ggcgtaatag 3540
   cgaagaggcc cgcaccgatc gcccttccca acagttgcgc agcctgaatg gcgaatggcg 3600
   cctgatgcgg tattttctcc ttacgcatct gtgcggtatt tcacaccgca tatggtgcac 3660
   tctcagtaca atctgctctg atgccgcata gttaagccag ccccgacacc cgccaacacc 3720
   cgctgacgcg ccctgacggg cttgtctgct cccggcatcc gcttacagac aagctgtgac 3780
   cgtctccggg agctgcatgt gtcagaggtt ttcaccgtca tcaccgaaac gcgcgagacg 3840
   aaagggcctc gtgatacgcc tatttttata ggttaatgtc atgataataa tggtttctta 3900
   gacgtcaggt ggcacttttc ggggaaatgt gcgcggaacc cctatttgtt tatttttcta 3960
   aatacattca aatatgtatc cgctcatgag acaataaccc tgataaatgc ttcaatggcg 4020
   cgccgcggcc gcttaagaat attgaaaaag gaagagtatg agtattcaac atttccgtgt 4080
   cgcccttatt cccttttttg cggcattttg ccttcctgtt tttgctcacc cagaaacgct 4140
   ggtgaaagta aaagatgctg aagatcagtt gggtgcacga gtgggttaca tcgaactgga 4200
   tctcaacagc ggtaagatcc ttgagagttt tcgccccgaa gaacgttttc caatgatgag 4260
   cacttttaaa gttctgctat gtggcgcggt attatcccgt attgacgccg ggcaagagca 4320
   actcggtcgc cgcatacact attctcagaa tgacttggtt gagtactcac cagtcacaga 4380
   aaagcatctt acggatggca tgacagtaag agaattatgc agtgctgcca taaccatgag 4440
   tgataacact gcggccaact tacttctgac aacgatcgga ggaccgaagg agctaaccgc 4500
   ttttttgcac aacatggggg atcatgtaac tcgccttgat cgttgggaac cggagctgaa 4560
   tgaagccata ccaaacgacg agcgtgacac cacgatgcct gtagcaatgg caacaacgtt 4620
   gcgcaaacta ttaactggcg aactacttac tctagcttcc cggcaacaat taatagactg 4680
   gatggaggcg gataaagttg caggaccact tctgcgctcg gcccttccgg ctggctggtt 4740
   tattgctgat aaatctggag ccggtgagcg tgggtctcgc ggtatcattg cagcactggg 4800
   gccagatggt aagccctccc gtatcgtagt tatctacacg acggggagtc aggcaactat 4860
   ggatgaacga aatagacaga tcgctgagat aggtgcctca ctgattaagc attggtaact 4920
   gtcagaccaa gtttactcat atatacttta gattgattta aaacttcatt tttaatttaa 4980
   aaggatctag gtgaagatcc tttttgataa tctcatgacc aaaatccctt aacgtgagtt 5040
   ttcgttccac tgagcgtcag accccgtaga aaagatcaaa ggatcttctt gagatccttt 5100
   ttttctgcgc gtaatctgct gcttgcaaac aaaaaaacca ccgctaccag cggtggtttg 5160
   tttgccggat caagagctac caactctttt tccgaaggta actggcttca gcagagcgca 5220
   gataccaaat actgtccttc tagtgtagcc gtagttaggc caccacttca agaactctgt 5280
   agcaccgcct acatacctcg ctctgctaat cctgttacca gtggctgctg ccagtggcga 5340
   taagtcgtgt cttaccgggt tggactcaag acgatagtta ccggataagg cgcagcggtc 5400
   gggctgaacg gggggttcgt gcacacagcc cagcttggag cgaacgacct acaccgaact 5460
   gagataccta cagcgtgagc tatgagaaag cgccacgctt cccgaaggga gaaaggcgga 5520
   caggtatccg gtaagcggca gggtcggaac aggagagcgc acgagggagc ttccaggggg 5580
   aaacgcctgg tatctttata gtcctgtcgg gtttcgccac ctctgacttg agcgtcgatt 5640
   tttgtgatgc tcgtcagggg ggcggagcct atggaaaaac gccagcaacg cggccttttt 5700
   acggttcctg gccttttgct ggccttttgc tcacatgttc tttcctgcgt tatcccctga 5760
   ttctgtggat aaccgtatta ccgcctttga gtgagctgat accgctcgcc gcagccgaac 5820
   gaccgagcgc agcgagtcag tgagcgagga agcggaagag cttaagcggc cgcggcgcgc 5880
   cgcccaatac gcaaaccgcc tctccccgcg cgttggccga ttcattaatg cagctggcac 5940
   gacaggtttc ccgactggaa agcgggcagt gagcgcaacg caattaatgt gagttagctc 6000
   actcattagg caccccaggc tttacacttt atgcttccgg ctcgtatgtt gtgtggaatt 6060
   gtgagcggat aacaatttca cacaggaaac agctatgacc atgattacgc c 6111
<210> 10
   <211> 13737
   <212> DNA
   <213> Plasmid
<220>
   <223> Description of Artificial Sequence:Plasmid
<400> 10
   gatccagaat tcgtgatcaa atggccgcaa caagcagcac aagcagccag tcttttgaca 60
   tagagctcga catcatcggc cagcaaccgc ctcttctttc aatctacacc cagatcagtc 120
   tcgtttaccc cgtctctgat ccctcccagt atcccaccat cgtcagcacc cttgaggaag 180
   gcctaaaacg cctctctcaa accttcccat gggtcgcggg ccaggtcaag accgagggca 240
   tcagcgaagg aaacacagga acttccaaga tcattccata tgaggagaca ccccgtcttg 300
   tggtgaaaga cctccgtgat gattcctcag cgccaacgat cgaggggttg agaaaggcgg 360
   gtttcccctt agagatgttt gacgagaacg tcgtcgctcc gaggaagaca ttagctatcg 420
   gacctggcaa tggccccaac gacccgaagc ctgtgttgct attgcagctc aacttcatta 480
   agggcggact cattctcacc gtcaacggac aacatggtgc tatggacatg acaggacaag 540
   atgcaattat tcgtcttctc tccaaggcgt gccgcaacga atcattcacc gaggaggaaa 600
   tctcggccat gaacctcgat cgcaagacgg tagtccctct ccttgaaaac tacaaagttg 660
   gtcctgagct agaccaccag atcgccaaac ctgcgcctgc tggcgacgct ccacccgcac 720
   cggccaaggc aagctgggcg ttcttttcat tcactcccaa ggccctctcg gagctgaaag 780
   acgcagccac aaagactctt gacgcgtcgt ccaagtttgt gtcaactgat gatgctcttt 840
   cggcgtttat ctggcaatca acctcgcgcg tacgtctcgc aagattggat gcttccacac 900
   ctactgaatt ctgccgcgct gtcgacatgc ggggcccaat gggcgtatca agcacatacc 960
   caggccttct tcaaaacatg acctaccatg actcgaccgt cgccgaaatc gccaacgaac 1020
   cacttggcgc aacagcatca cgcctgcgct cggaactcaa cagtgatcgt ttgcgcagac 1080
   gaacacaagc tttggcgacg tacatgcatg gcctgcctga caagtcgagc gtctccctga 1140
   ccgccgatgc gaatccgtca agcagcatca tgctgagttc ctgggccaag gtgggatgct 1200
   gggagtatga ctttgggttt ggactgggta agcctgagag tgtgagaaga cctcgctttg 1260
   aacctttgga gagtttgatg tactttatgc ccaagaagcc tgatggggag tttacggcgt 1320
   ccatttctct gagggatgag gatatggaga gactaaaggc ggatgaggag tggacaaagt 1380
   acgcaaagta tattgggtag atagtttact agactactgc agggatatcg tggatccccc 1440
   gaatttcccc gatcgttcaa acatttggca ataaagtttc ttaagattga atcctgttgc 1500
   cggtcttgcg atgattatca tctaatttct gttgaattac gttaagcatg taataattaa 1560
   catgtaatgc atgacgttat ttatgagatg ggtttttatg attagagtcc cgcaattata 1620
   catttaatac gcgatagaaa acaaaatata gcgcgcaaac taggataaat tatcgcgcgc 1680
   ggtgtcatct atgttactag atccgggaat tcggcgcgcc caattgattt aaatggccgc 1740
   tgcggccaat tcctgcagcg ttgcggttct gtcagttcca aacgtaaaac ggcttgtccc 1800
   gcgtcatcgg cgggggtcat aacgtgactc ccttaattct ccgctcatga tcagattgtc 1860
   gtttcccgcc ttcagtttaa actatcagtg tttgacagga tatattggcg ggtaaaccta 1920
   agagaaaaga gcgtttatta gaataatcgg atatttaaaa gggcgtgaaa aggtttatcc 1980
   gttcgtccat ttgtatgtgc atgccaacca cagggttccc cagatctggc gccggccagc 2040
   gagacgagca agattggccg ccgcccgaaa cgatccgaca gcgcgcccag cacaggtgcg 2100
   caggcaaatt gcaccaacgc atacagcgcc agcagaatgc catagtgggc ggtgacgtcg 2160
   ttcgagtgaa ccagatcgcg caggaggccc ggcagcaccg gcataatcag gccgatgccg 2220
   acagcgtcga gcgcgacagt gctcagaatt acgatcaggg gtatgttggg tttcacgtct 2280
   ggcctccgga ccagcctccg ctggtccgat tgaacgcgcg gattctttat cactgataag 2340
   ttggtggaca tattatgttt atcagtgata aagtgtcaag catgacaaag ttgcagccga 2400
   atacagtgat ccgtgccgcc ctggacctgt tgaacgaggt cggcgtagac ggtctgacga 2460
   cacgcaaact ggcggaacgg ttgggggttc agcagccggc gctttactgg cacttcagga 2520
   acaagcgggc gctgctcgac gcactggccg aagccatgct ggcggagaat catacgcatt 2580
   cggtgccgag agccgacgac gactggcgct catttctgat cgggaatgcc cgcagcttca 2640
   ggcaggcgct gctcgcctac cgcgatggcg cgcgcatcca tgccggcacg cgaccgggcg 2700
   caccgcagat ggaaacggcc gacgcgcagc ttcgcttcct ctgcgaggcg ggtttttcgg 2760
   ccggggacgc cgtcaatgcg ctgatgacaa tcagctactt cactgttggg gccgtgcttg 2820
   aggagcaggc cggcgacagc gatgccggcg agcgcggcgg caccgttgaa caggctccgc 2880
   tctcgccgct gttgcgggcc gcgatagacg ccttcgacga agccggtccg gacgcagcgt 2940
   tcgagcaggg actcgcggtg attgtcgatg gattggcgaa aaggaggctc gttgtcagga 3000
   acgttgaagg accgagaaag ggtgacgatt gatcaggacc gctgccggag cgcaacccac 3060
   tcactacagc agagccatgt agacaacatc ccctccccct ttccaccgcg tcagacgccc 3120
   gtagcagccc gctacgggct ttttcatgcc ctgccctagc gtccaagcct cacggccgcg 3180
   ctcggcctct ctggcggcct tctggcgctc ttccgcttcc tcgctcactg actcgctgcg 3240
   ctcggtcgtt cggctgcggc gagcggtatc agctcactca aaggcggtaa tacggttatc 3300
   cacagaatca ggggataacg caggaaagaa catgtgagca aaaggccagc aaaaggccag 3360
   gaaccgtaaa aaggccgcgt tgctggcgtt tttccatagg ctccgccccc ctgacgagca 3420
   tcacaaaaat cgacgctcaa gtcagaggtg gcgaaacccg acaggactat aaagatacca 3480
   ggcgtttccc cctggaagct ccctcgtgcg ctctcctgtt ccgaccctgc cgcttaccgg 3540
   atacctgtcc gcctttctcc cttcgggaag cgtggcgctt ttccgctgca taaccctgct 3600
   tcggggtcat tatagcgatt ttttcggtat atccatcctt tttcgcacga tatacaggat 3660
   tttgccaaag ggttcgtgta gactttcctt ggtgtatcca acggcgtcag ccgggcagga 3720
   taggtgaagt aggcccaccc gcgagcgggt gttccttctt cactgtccct tattcgcacc 3780
   tggcggtgct caacgggaat cctgctctgc gaggctggcc ggctaccgcc ggcgtaacag 3840
   atgagggcaa gcggatggct gatgaaacca agccaaccag gaagggcagc ccacctatca 3900
   aggtgtactg ccttccagac gaacgaagag cgattgagga aaaggcggcg gcggccggca 3960
   tgagcctgtc ggcctacctg ctggccgtcg gccagggcta caaaatcacg ggcgtcgtgg 4020
   actatgagca cgtccgcgag ctggcccgca tcaatggcga cctgggccgc ctgggcggcc 4080
   tgctgaaact ctggctcacc gacgacccgc gcacggcgcg gttcggtgat gccacgatcc 4140
   tcgccctgct ggcgaagatc gaagagaagc aggacgagct tggcaaggtc atgatgggcg 4200
   tggtccgccc gagggcagag ccatgacttt tttagccgct aaaacggccg gggggtgcgc 4260
   gtgattgcca agcacgtccc catgcgctcc atcaagaaga gcgacttcgc ggagctggtg 4320
   aagtacatca ccgacgagca aggcaagacc gagcgccttt gcgacgctca ccgggctggt 4380
   tgccctcgcc gctgggctgg cggccgtcta tggccctgca aacgcgccag aaacgccgtc 4440
   gaagccgtgt gcgagacacc gcggccggcc gccggcgttg tggatacctc gcggaaaact 4500
   tggccctcac tgacagatga ggggcggacg ttgacacttg aggggccgac tcacccggcg 4560
   cggcgttgac agatgagggg caggctcgat ttcggccggc gacgtggagc tggccagcct 4620
   cgcaaatcgg cgaaaacgcc tgattttacg cgagtttccc acagatgatg tggacaagcc 4680
   tggggataag tgccctgcgg tattgacact tgaggggcgc gactactgac agatgagggg 4740
   cgcgatcctt gacacttgag gggcagagtg ctgacagatg aggggcgcac ctattgacat 4800
   ttgaggggct gtccacaggc agaaaatcca gcatttgcaa gggtttccgc ccgtttttcg 4860
   gccaccgcta acctgtcttt taacctgctt ttaaaccaat atttataaac cttgttttta 4920
   accagggctg cgccctgtgc gcgtgaccgc gcacgccgaa gggggtgccc cccccttctc 4980
   gaaccctccc ggcccgctaa cgcgggcctc ccatcccccc aggggctgcg cccctcggcc 5040
   gcgaacggcc tcaccccaaa aatggcagcg ctggcagtcc ttgccattgc cgggatcggg 5100
   gcagtaacgg gatgggcgat cagcccgagc gcgacgcccg gaagcattga cgtgccgcag 5160
   gtgctggcat cgacattcag cgaccaggtg ccgggcagtg agggcggcgg cctgggtggc 5220
   ggcctgccct tcacttcggc cgtcggggca ttcacggact tcatggcggg gccggcaatt 5280
   tttaccttgg gcattcttgg catagtggtc gcgggtgccg tgctcgtgtt cgggggtgcg 5340
   ataaacccag cgaaccattt gaggtgatag gtaagattat accgaggtat gaaaacgaga 5400
   attggacctt tacagaatta ctctatgaag cgccatattt aaaaagctac caagacgaag 5460
   aggatgaaga ggatgaggag gcagattgcc ttgaatatat tgacaatact gataagataa 5520
   tatatctttt atatagaaga tatcgccgta tgtaaggatt tcagggggca aggcataggc 5580
   agcgcgctta tcaatatatc tatagaatgg gcaaagcata aaaacttgca tggactaatg 5640
   cttgaaaccc aggacaataa ccttatagct tgtaaattct atcataattg ggtaatgact 5700
   ccaacttatt gatagtgttt tatgttcaga taatgcccga tgactttgtc atgcagctcc 5760
   accgattttg agaacgacag cgacttccgt cccagccgtg ccaggtgctg cctcagattc 5820
   aggttatgcc gctcaattcg ctgcgtatat cgcttgctga ttacgtgcag ctttcccttc 5880
   aggcgggatt catacagcgg ccagccatcc gtcatccata tcaccacgtc aaagggtgac 5940
   agcaggctca taagacgccc cagcgtcgcc atagtgcgtt caccgaatac gtgcgcaaca 6000
   accgtcttcc ggagactgtc atacgcgtaa aacagccagc gctggcgcga tttagccccg 6060
   acatagcccc actgttcgtc catttccgcg cagacgatga cgtcactgcc cggctgtatg 6120
   cgcgaggtta ccgactgcgg cctgagtttt ttaagtgacg taaaatcgtg ttgaggccaa 6180
   cgcccataat gcgggctgtt gcccggcatc caacgccatt catggccata tcaatgattt 6240
   tctggtgcgt accgggttga gaagcggtgt aagtgaactg cagttgccat gttttacggc 6300
   agtgagagca gagatagcgc tgatgtccgg cggtgctttt gccgttacgc accaccccgt 6360
   cagtagctga acaggaggga cagctgatag acacagaagc cactggagca cctcaaaaac 6420
   accatcatac actaaatcag taagttggca gcatcaccca taattgtggt ttcaaaatcg 6480
   gctccgtcga tactatgtta tacgccaact ttgaaaacaa ctttgaaaaa gctgttttct 6540
   ggtatttaag gttttagaat gcaaggaaca gtgaattgga gttcgtcttg ttataattag 6600
   cttcttgggg tatctttaaa tactgtagaa aagaggaagg aaataataaa tggctaaaat 6660
   gagaatatca ccggaattga aaaaactgat cgaaaaatac cgctgcgtaa aagatacgga 6720
   aggaatgtct cctgctaagg tatataagct ggtgggagaa aatgaaaacc tatatttaaa 6780
   aatgacggac agccggtata aagggaccac ctatgatgtg gaacgggaaa aggacatgat 6840
   gctatggctg gaaggaaagc tgcctgttcc aaaggtcctg cactttgaac ggcatgatgg 6900
   ctggagcaat ctgctcatga gtgaggccga tggcgtcctt tgctcggaag agtatgaaga 6960
   tgaacaaagc cctgaaaaga ttatcgagct gtatgcggag tgcatcaggc tctttcactc 7020
   catcgacata tcggattgtc cctatacgaa tagcttagac agccgcttag ccgaattgga 7080
   ttacttactg aataacgatc tggccgatgt ggattgcgaa aactgggaag aagacactcc 7140
   atttaaagat ccgcgcgagc tgtatgattt tttaaagacg gaaaagcccg aagaggaact 7200
   tgtcttttcc cacggcgacc tgggagacag caacatcttt gtgaaagatg gcaaagtaag 7260
   tggctttatt gatcttggga gaagcggcag ggcggacaag tggtatgaca ttgccttctg 7320
   cgtccggtcg atcagggagg atatcgggga agaacagtat gtcgagctat tttttgactt 7380
   actggggatc aagcctgatt gggagaaaat aaaatattat attttactgg atgaattgtt 7440
   ttagtaccta gatgtggcgc aacgatgccg gcgacaagca ggagcgcacc gacttcttcc 7500
   gcatcaagtg ttttggctct caggccgagg cccacggcaa gtatttgggc aaggggtcgc 7560
   tggtattcgt gcagggcaag attcggaata ccaagtacga gaaggacggc cagacggtct 7620
   acgggaccga cttcattgcc gataaggtgg attatctgga caccaaggca ccaggcgggt 7680
   caaatcagga ataagggcac attgccccgg cgtgagtcgg ggcaatcccg caaggagggt 7740
   gaatgaatcg gacgtttgac cggaaggcat acaggcaaga actgatcgac gcggggtttt 7800
   ccgccgagga tgccgaaacc atcgcaagcc gcaccgtcat gcgtgcgccc cgcgaaacct 7860
   tccagtccgt cggctcgatg gtccagcaag ctacggccaa gatcgagcgc gacagcgtgc 7920
   aactggctcc ccctgccctg cccgcgccat cggccgccgt ggagcgttcg cgtcgtctcg 7980
   aacaggaggc ggcaggtttg gcgaagtcga tgaccatcga cacgcgagga actatgacga 8040
   ccaagaagcg aaaaaccgcc ggcgaggacc tggcaaaaca ggtcagcgag gccaagcagg 8100
   ccgcgttgct gaaacacacg aagcagcaga tcaaggaaat gcagctttcc ttgttcgata 8160
   ttgcgccgtg gccggacacg atgcgagcga tgccaaacga cacggcccgc tctgccctgt 8220
   tcaccacgcg caacaagaaa atcccgcgcg aggcgctgca aaacaaggtc attttccacg 8280
   tcaacaagga cgtgaagatc acctacaccg gcgtcgagct gcgggccgac gatgacgaac 8340
   tggtgtggca gcaggtgttg gagtacgcga agcgcacccc tatcggcgag ccgatcacct 8400
   tcacgttcta cgagctttgc caggacctgg gctggtcgat caatggccgg tattacacga 8460
   aggccgagga atgcctgtcg cgcctacagg cgacggcgat gggcttcacg tccgaccgcg 8520
   ttgggcacct ggaatcggtg tcgctgctgc accgcttccg cgtcctggac cgtggcaaga 8580
   aaacgtcccg ttgccaggtc ctgatcgacg aggaaatcgt cgtgctgttt gctggcgacc 8640
   actacacgaa attcatatgg gagaagtacc gcaagctgtc gccgacggcc cgacggatgt 8700
   tcgactattt cagctcgcac cgggagccgt acccgctcaa gctggaaacc ttccgcctca 8760
   tgtgcggatc ggattccacc cgcgtgaaga agtggcgcga gcaggtcggc gaagcctgcg 8820
   aagagttgcg aggcagcggc ctggtggaac acgcctgggt caatgatgac ctggtgcatt 8880
   gcaaacgcta gggccttgtg gggtcagttc cggctggggg ttcagcagcc agcgctttac 8940
   tggcatttca ggaacaagcg ggcactgctc gacgcacttg cttcgctcag tatcgctcgg 9000
   gacgcacggc gcgctctacg aactgccgat aaacagagga ttaaaattga caattgtgat 9060
   taaggctcag attcgacggc ttggagcggc cgacgtgcag gatttccgcg agatccgatt 9120
   gtcggccctg aagaaagctc cagagatgtt cgggtccgtt tacgagcacg aggagaaaaa 9180
   gcccatggag gcgttcgctg aacggttgcg agatgccgtg gcattcggcg cctacatcga 9240
   cggcgagatc attgggctgt cggtcttcaa acaggaggac ggccccaagg acgctcacaa 9300
   ggcgcatctg tccggcgttt tcgtggagcc cgaacagcga ggccgagggg tcgccggtat 9360
   gctgctgcgg gcgttgccgg cgggtttatt gctcgtgatg atcgtccgac agattccaac 9420
   gggaatctgg tggatgcgca tcttcatcct cggcgcactt aatatttcgc tattctggag 9480
   cttgttgttt atttcggtct accgcctgcc gggcggggtc gcggcgacgg taggcgctgt 9540
   gcagccgctg atggtcgtgt tcatctctgc cgctctgcta ggtagcccga tacgattgat 9600
   ggcggtcctg gggctatttt gcggaactgc gggcgtggcg ctgttggtgt tgacaccaaa 9660
   cgcagcgcta gatcctgtcg gcgtcgcagc gggcctggcg gggcggtttc ccatggcgtt 9720
   cggaaccgtg ctgacccgca agtggcaacc tcccgtgcct ctgctcacct ttaccgcctg 9780
   gcaactggcg gccggaggac ttctgctcgt tccagtagct ttagtgtttg atccgccaat 9840
   cccgatgcct acaggaacca atgttctcgg cctggcgtgg ctcggcctga tcggagcggg 9900
   tttaacctac ttcctttggt tccgggggat ctcgcgactc gaacctacag ttgtttcctt 9960
   actgggcttt ctcagcccca gatctggggt cgatcagccg gggatgcatc aggccgacag 10020
   tcggaacttc gggtccccga cctgtaccat tcggtgagca atggataggg gagttgatat 10080
   cgtcaacgtt cacttctaaa gaaatagcgc cactcagctt cctcagcggc tttatccagc 10140
   gatttcctat tatgtcggca tagttctcaa gatcgacagc ctgtcacggt taagcgagaa 10200
   atgaataaga aggctgataa ttcggatctc tgcgagggag atgatatttg atcacaggca 10260
   gcaacgctct gtcatcgtta caatcaacat gctaccctcc gcgagatcat ccgtgtttca 10320
   aacccggcag cttagttgcc gttcttccga atagcatcgg taacatgagc aaagtctgcc 10380
   gccttacaac ggctctcccg ctgacgccgt cccggactga tgggctgcct gtatcgagtg 10440
   gtgattttgt gccgagctgc cggtcgggga gctgttggct ggctggtggc aggatatatt 10500
   gtggtgtaaa caaattgacg cttagacaac ttaataacac attgcggacg tttttaatgt 10560
   actgcggtac ggccatgctg gccgcccggg caccggtaaa tttcctgcag ggctagcgaa 10620
   ttcgagctcg gtacccctgg attttggttt taggaattag attattgata gaagtatttt 10680
   acaaatacaa atacatacta agggtttctt atatgctcaa cacatgagcg aaaccctata 10740
   agaaccctaa ttcccttatc tgggaactac tcacacatta ttatagagag agatagattt 10800
   gtagagagag actggtgatt tcagcgggca tgcctgcagg tcgactcaga tctgggtaac 10860
   tggcctaact ggccttggag gagctggcaa ctcaaaatcc ctttgccaaa aaccaacatc 10920
   atgccatcca ccatgcttgt atccagctgc gcgcaatgta ccccgggctg tgtatcccaa 10980
   agcctcatgc aacctaacag atggatcgtt tggaaggcct ataacagcaa ccacagactt 11040
   aaaaccttgc gcctccatag acttaagcaa atgtgtgtac aatgtggatc ctaggcccaa 11100
   cctttgatgc ctatgtgaca cgtaaacagt actctcaact gtccaatcgt aagcgttcct 11160
   agccttccag ggcccagcgt aagcaatacc agccacaaca ccctcaacct cagcaaccaa 11220
   ccaagggtat ctatcttgca acctctctag atcatcaatc cactcttgtg gtgtttgtgg 11280
   ctctgtccta aagttcactg tagacgtctc aatgtaatgg ttaacgatat cacaaaccgc 11340
   ggccatatca gctgctgtag ctggcctaat ctcaactggt ctcctctccg gagacatgtc 11400
   gactctagag gatccccggg taccctgtcc tctccaaatg aaatgaactt ccttatatag 11460
   aggaagggtc ttgcgaagga tagtgggatt gtgcgtcatc ccttacgtca gtggagatat 11520
   cacatcaatc cacttgcttt gaagacgtgg ttggaacgtc ttctttttcc acgatgctcc 11580
   tcgtgggtgg gggtccatct ttgggaccac tgtcggcaga ggcatcttca acgatggcct 11640
   ttcctttatc gcaatgatgg catttgtagg agccaccttc cttttccact atcttcacaa 11700
   taaagtgaca gatagctggg caatggaatc cgaggaggtt tccggatatt accctttgtt 11760
   gaaaagtctc aattgccctt tggtcttctg agactgtatc tttgatattt ttggagtaga 11820
   caagcgtgtc gtgctccacc atgttgacga agatattctt cttgtcattg agtcgtaaga 11880
   gactctgtat gaactgttcg ccagtcttta cggcgagttc tgttggtcct ctatttgaat 11940
   ctttgactcc atgggaattg agatctctcg aggtttaaac gggccacgcc tgcggccgcc 12000
   tcgaggtacc ggatttggag ccaagtctca taaacgccat tgtggaagaa agtcttgagt 12060
   tggtggtaat gtaacagagt agtaagaaca gagaagagag agagtgtgag atacatgaat 12120
   tgtcgggcaa caaaaatcct gaacatctta ttttagcaaa gagaaagagt tccgagtctg 12180
   tagcagaaga gtgaggagaa atttaagctc ttggacttgt gaattgttcc gcctcttgaa 12240
   tacttcttca atcctcatat attcttcttc tatgttacct gaaaaccggc atttaatctc 12300
   gcgggtttat tccggttcaa catttttttt gttttgagtt attatctggg cttaataacg 12360
   caggcctgaa ataaattcaa ggcccaactg tttttttttt taagaagttg ctgttaaaaa 12420
   aaaaaaaagg gaattaacaa caacaacaaa aaaagataaa gaaaataata acaattactt 12480
   taattgtaga ctaaaaaaac atagatttta tcatgaaaaa aagagaaaag aaataaaaac 12540
   ttggatcaaa aaaaaaaaca tacagatctt ctaattatta acttttctta aaaattaggt 12600
   cctttttccc aacaattagg tttagagttt tggaattaaa ccaaaaagat tgttctaaaa 12660
   aatactcaaa tttggtagat aagtttcctt attttaatta gtcaatggta gatacttttt 12720
   tttcttttct ttattagagt agattagaat cttttatgcc aagttttgat aaattaaatc 12780
   aagaagataa actatcataa tcaacatgaa attaaaagaa aaatctcata tatagtatta 12840
   gtattctcta tatatattat gattgcttat tcttaatggg ttgggttaac caagacatag 12900
   tcttaatgga aagaatcttt tttgaacttt ttccttattg attaaattct tctatagaaa 12960
   agaaagaaat tatttgagga aaagtatata caaaaagaaa aatagaaaaa tgtcagtgaa 13020
   gcagatgtaa tggatgacct aatccaacca ccaccatagg atgtttctac ttgagtcggt 13080
   cttttaaaaa cgcacggtgg aaaatatgac acgtatcata tgattccttc ctttagtttc 13140
   gtgataataa tcctcaactg atatcttcct ttttttgttt tggctaaaga tattttattc 13200
   tcattaatag aaaagacggt tttgggcttt tggtttgcga tataaagaag accttcgtgt 13260
   ggaagataat aattcatcct ttcgtctttt tctgactctt caatctctcc caaagcctaa 13320
   agcgatctct gcaaatctct cgcgactctc tctttcaagg tatattttct gattcttttt 13380
   gtttttgatt cgtatctgat ctccaatttt tgttatgtgg attattgaat cttttgtata 13440
   aattgctttt gacaatattg ttcgtttcgt caatccagct tctaaatttt gtcctgatta 13500
   ctaagatatc gattcgtagt gtttacatct gtgtaatttc ttgcttgatt gtgaaattag 13560
   gattttcaag gacgatctat tcaatttttg tgttttcttt gttcgattct ctctgtttta 13620
   ggtttcttat gtttagatcc gtttctcttt ggtgttgttt tgatttctct tacggctttt 13680
   gatttggtat atgttcgctg attggtttct acttgttcta ttgttttatt tcaggtg    13737
<210> 11
   <211> 12949
   <212> DNA
   <213> Plasmid
<400> 11
   agcttgcatg cctgcagtgc agcgtgaccc ggtcgtgccc ctctctagag ataatgagca 60
   ttgcatgtct aagttataaa aaattaccac atattttttt tgtcacactt gtttgaagtg 120
   cagtttatct atctttatac atatatttaa actttactct acgaataata taatctatag 180
   tactacaata atatcagtgt tttagagaat catataaatg aacagttaga catggtctaa 240
   aggacaattg agtattttga caacaggact ctacagtttt atctttttag tgtgcatgtg 300
   ttctcctttt tttttgcaaa tagcttcacc tatataatac ttcatccatt ttattagtac 360
   atccatttag ggtttagggt taatggtttt tatagactaa tttttttagt acatctattt 420
   tattctattt tagcctctaa attaagaaaa ctaaaactct attttagttt ttttatttaa 480
   taatttagat ataaaataga ataaaataaa gtgactaaaa attaaacaaa taccctttaa 540
   gaaattaaaa aaactaagga aacatttttc ttgtttcgag tagataatgc cagcctgtta 600
   aacgccgtcg acgagtctaa cggacaccaa ccagcgaacc agcagcgtcg cgtcgggcca 660
   agcgaagcag acggcacggc atctctgtcg ctgcctctgg acccctctcg agagttccgc 720
   tccaccgttg gacttgctcc gctgtcggca tccagaaatt gcgtggcgga gcggcagacg 780
   tgagccggca cggcaggcgg cctcctcctc ctctcacggc accggcagct acgggggatt 840
   cctttcccac cgctccttcg ctttcccttc ctcgcccgcc gtaataaata gacaccccct 900
   ccacaccctc tttccccaac ctcgtgttgt tcggagcgca cacacacaca accagatctc 960
   ccccaaatcc acccgtcggc acctccgctt caaggtacgc cgctcgtcct cccccccccc 1020
   ccctctctac cttctctaga tcggcgttcc ggtccatggt tagggcccgg tagttctact 1080
   tctgttcatg tttgtgttag atccgtgttt gtgttagatc cgtgctgcta gcgttcgtac 1140
   acggatgcga cctgtacgtc agacacgttc tgattgctaa cttgccagtg tttctctttg 1200
   gggaatcctg ggatggctct agccgttccg cagacgggat cgatttcatg attttttttg 1260
   tttcgttgca tagggtttgg tttgcccttt tcctttattt caatatatgc cgtgcacttg 1320
   tttgtcgggt catcttttca tgcttttttt tgtcttggtt gtgatgatgt ggtctggttg 1380
   ggcggtcgtt ctagatcgga gtagaattct gtttcaaact acctggtgga tttattaatt 1440
   ttggatctgt atgtgtgtgc catacatatt catagttacg aattgaagat gatggatgga 1500
   aatatcgatc taggataggt atacatgttg atgcgggttt tactgatgca tatacagaga 1560
   tgctttttgt tcgcttggtt gtgatgatgt ggtgtggttg ggcggtcgtt cattcgttct 1620
   agatcggagt agaatactgt ttcaaactac ctggtgtatt tattaatttt ggaactgtat 1680
   gtgtgtgtca tacatcttca tagttacgag tttaagatgg atggaaatat cgatctagga 1740
   taggtataca tgttgatgtg ggttttactg atgcatatac atgatggcat atgcagcatc 1800
   tattcatatg ctctaacctt gagtacctat ctattataat aaacaagtat gttttataat 1860
   tattttgatc ttgatatact tggatgatgg catatgcagc agctatatgt ggattttttt 1920
   agccctgcct tcatacgcta tttatttgct tggtactgtt tcttttgtcg atgctcaccc 1980
   tgttgtttgg tgttacttct gcagggatcc ccgatcatgc aaaaactcat taactcagtg 2040
   caaaactatg cctggggcag caaaacggcg ttgactgaac tttatggtat ggaaaatccg 2100
   tccagccagc cgatggccga gctgtggatg ggcgcacatc cgaaaagcag ttcacgagtg 2160
   cagaatgccg ccggagatat cgtttcactg cgtgatgtga ttgagagtga taaatcgact 2220
   ctgctcggag aggccgttgc caaacgcttt ggcgaactgc ctttcctgtt caaagtatta 2280
   tgcgcagcac agccactctc cattcaggtt catccaaaca aacacaattc tgaaatcggt 2340
   tttgccaaag aaaatgccgc aggtatcccg atggatgccg ccgagcgtaa ctataaagat 2400
   cctaaccaca agccggagct ggtttttgcg ctgacgcctt tccttgcgat gaacgcgttt 2460
   cgtgaatttt ccgagattgt ctccctactc cagccggtcg caggtgcaca tccggcgatt 2520
   gctcactttt tacaacagcc tgatgccgaa cgtttaagcg aactgttcgc cagcctgttg 2580
   aatatgcagg gtgaagaaaa atcccgcgcg ctggcgattt taaaatcggc cctcgatagc 2640
   cagcagggtg aaccgtggca aacgattcgt ttaatttctg aattttaccc ggaagacagc 2700
   ggtctgttct ccccgctatt gctgaatgtg gtgaaattga accctggcga agcgatgttc 2760
   ctgttcgctg aaacaccgca cgcttacctg caaggcgtgg cgctggaagt gatggcaaac 2820
   tccgataacg tgctgcgtgc gggtctgacg cctaaataca ttgatattcc ggaactggtt 2880
   gccaatgtga aattcgaagc caaaccggct aaccagttgt tgacccagcc ggtgaaacaa 2940
   ggtgcagaac tggacttccc gattccagtg gatgattttg ccttctcgct gcatgacctt 3000
   agtgataaag aaaccaccat tagccagcag agtgccgcca ttttgttctg cgtcgaaggc 3060
   gatgcaacgt tgtggaaagg ttctcagcag ttacagctta aaccgggtga atcagcgttt 3120
   attgccgcca acgaatcacc ggtgactgtc aaaggccacg gccgtttagc gcgtgtttac 3180
   aacaagctgt aagagcttac tgaaaaaatt aacatctctt gctaagctgg gagctcgatc 3240
   cgtcgacctg cagatcgttc aaacatttgg caataaagtt tcttaagatt gaatcctgtt 3300
   gccggtcttg cgatgattat catataattt ctgttgaatt acgttaagca tgtaataatt 3360
   aacatgtaat gcatgacgtt atttatgaga tgggttttta tgattagagt cccgcaatta 3420
   tacatttaat acgcgataga aaacaaaata tagcgcgcaa actaggataa attatcgcgc 3480
   gcggtgtcat ctatgttact agatctgcta gccctgcagg aaatttaccg gtgcccgggc 3540
   ggccagcatg gccgtatccg caatgtgtta ttaagttgtc taagcgtcaa tttgtttaca 3600
   ccacaatata tcctgccacc agccagccaa cagctccccg accggcagct cggcacaaaa 3660
   tcaccactcg atacaggcag cccatcagaa ttaattctca tgtttgacag cttatcatcg 3720
   actgcacggt gcaccaatgc ttctggcgtc aggcagccat cggaagctgt ggtatggctg 3780
   tgcaggtcgt aaatcactgc ataattcgtg tcgctcaagg cgcactcccg ttctggataa 3840
   tgttttttgc gccgacatca taacggttct ggcaaatatt ctgaaatgag ctgttgacaa 3900
   ttaatcatcc ggctcgtata atgtgtggaa ttgtgagcgg ataacaattt cacacaggaa 3960
   acagaccatg agggaagcgt tgatcgccga agtatcgact caactatcag aggtagttgg 4020
   cgtcatcgag cgccatctcg aaccgacgtt gctggccgta catttgtacg gctccgcagt 4080
   ggatggcggc ctgaagccac acagtgatat tgatttgctg gttacggtga ccgtaaggct 4140
   tgatgaaaca acgcggcgag ctttgatcaa cgaccttttg gaaacttcgg cttcccctgg 4200
   agagagcgag attctccgcg ctgtagaagt caccattgtt gtgcacgacg acatcattcc 4260
   gtggcgttat ccagctaagc gcgaactgca atttggagaa tggcagcgca atgacattct 4320
   tgcaggtatc ttcgagccag ccacgatcga cattgatctg gctatcttgc tgacaaaagc 4380
   aagagaacat agcgttgcct tggtaggtcc agcggcggag gaactctttg atccggttcc 4440
   tgaacaggat ctatttgagg cgctaaatga aaccttaacg ctatggaact cgccgcccga 4500
   ctgggctggc gatgagcgaa atgtagtgct tacgttgtcc cgcatttggt acagcgcagt 4560
   aaccggcaaa atcgcgccga aggatgtcgc tgccgactgg gcaatggagc gcctgccggc 4620
   ccagtatcag cccgtcatac ttgaagctag gcaggcttat cttggacaag aagatcgctt 4680
   ggcctcgcgc gcagatcagt tggaagaatt tgttcactac gtgaaaggcg agatcaccaa 4740
   agtagtcggc aaataaagct ctagtggatc tccgtacccc cgggggatct ggctcgcggc 4800
   ggacgcacga cgccggggcg agaccatagg cgatctccta aatcaatagt agctgtaacc 4860
   tcgaagcgtt tcacttgtaa caacgattga gaatttttgt cataaaattg aaatacttgg 4920
   ttcgcatttt tgtcatccgc ggtcagccgc aattctgacg aactgcccat ttagctggag 4980
   atgattgtac atccttcacg tgaaaatttc tcaagcgctg tgaacaaggg ttcagatttt 5040
   agattgaaag gtgagccgtt gaaacacgtt cttcttgtcg atgacgacgt cgctatgcgg 5100
   catcttatta ttgaatacct tacgatccac gccttcaaag tgaccgcggt agccgacagc 5160
   acccagttca caagagtact ctcttccgcg acggtcgatg tcgtggttgt tgatctaaat 5220
   ttaggtcgtg aagatgggct cgagatcgtt cgtaatctgg cggcaaagtc tgatattcca 5280
   atcataatta tcagtggcga ccgccttgag gagacggata aagttgttgc actcgagcta 5340
   ggagcaagtg attttatcgc taagccgttc agtatcagag agtttctagc acgcattcgg 5400
   gttgccttgc gcgtgcgccc caacgttgtc cgctccaaag accgacggtc tttttgtttt 5460
   actgactgga cacttaatct caggcaacgt cgcttgatgt ccgaagctgg cggtgaggtg 5520
   aaacttacgg caggtgagtt caatcttctc ctcgcgtttt tagagaaacc ccgcgacgtt 5580
   ctatcgcgcg agcaacttct cattgccagt cgagtacgcg acgaggaggt ttatgacagg 5640
   agtatagatg ttctcatttt gaggctgcgc cgcaaacttg aggcagatcc gtcaagccct 5700
   caactgataa aaacagcaag aggtgccggt tatttctttg acgcggacgt gcaggtttcg 5760
   cacgggggga cgatggcagc ctgagccaat tcccagatcc ccgaggaatc ggcgtgagcg 5820
   gtcgcaaacc atccggcccg gtacaaatcg gcgcggcgct gggtgatgac ctggtggaga 5880
   agttgaaggc cgcgcaggcc gcccagcggc aacgcatcga ggcagaagca cgccccggtg 5940
   aatcgtggca agcggccgct gatcgaatcc gcaaagaatc ccggcaaccg ccggcagccg 6000
   gtgcgccgtc gattaggaag ccgcccaagg gcgacgagca accagatttt ttcgttccga 6060
   tgctctatga cgtgggcacc cgcgatagtc gcagcatcat ggacgtggcc gttttccgtc 6120
   tgtcgaagcg tgaccgacga gctggcgagg tgatccgcta cgagcttcca gacgggcacg 6180
   tagaggtttc cgcagggccg gccggcatgg ccagtgtgtg ggattacgac ctggtactga 6240
   tggcggtttc ccatctaacc gaatccatga accgataccg ggaagggaag ggagacaagc 6300
   ccggccgcgt gttccgtcca cacgttgcgg acgtactcaa gttctgccgg cgagccgatg 6360
   gcggaaagca gaaagacgac ctggtagaaa cctgcattcg gttaaacacc acgcacgttg 6420
   ccatgcagcg tacgaagaag gccaagaacg gccgcctggt gacggtatcc gagggtgaag 6480
   ccttgattag ccgctacaag atcgtaaaga gcgaaaccgg gcggccggag tacatcgaga 6540
   tcgagctagc tgattggatg taccgcgaga tcacagaagg caagaacccg gacgtgctga 6600
   cggttcaccc cgattacttt ttgatcgatc ccggcatcgg ccgttttctc taccgcctgg 6660
   cacgccgcgc cgcaggcaag gcagaagcca gatggttgtt caagacgatc tacgaacgca 6720
   gtggcagcgc cggagagttc aagaagttct gtttcaccgt gcgcaagctg atcgggtcaa 6780
   atgacctgcc ggagtacgat ttgaaggagg aggcggggca ggctggcccg atcctagtca 6840
   tgcgctaccg caacctgatc gagggcgaag catccgccgg ttcctaatgt acggagcaga 6900
   tgctagggca aattgcccta gcaggggaaa aaggtcgaaa aggtctcttt cctgtggata 6960
   gcacgtacat tgggaaccca aagccgtaca ttgggaaccg gaacccgtac attgggaacc 7020
   caaagccgta cattgggaac cggtcacaca tgtaagtgac tgatataaaa gagaaaaaag 7080
   gcgatttttc cgcctaaaac tctttaaaac ttattaaaac tcttaaaacc cgcctggcct 7140
   gtgcataact gtctggccag cgcacagccg aagagctgca aaaagcgcct acccttcggt 7200
   cgctgcgctc cctacgcccc gccgcttcgc gtcggcctat cgcggccgct ggccgctcaa 7260
   aaatggctgg cctacggcca ggcaatctac cagggcgcgg acaagccgcg ccgtcgccac 7320
   tcgaccgccg gcgctgaggt ctgcctcgtg aagaaggtgt tgctgactca taccaggcct 7380
   gaatcgcccc atcatccagc cagaaagtga gggagccacg gttgatgaga gctttgttgt 7440
   aggtggacca gttggtgatt ttgaactttt gctttgccac ggaacggtct gcgttgtcgg 7500
   gaagatgcgt gatctgatcc ttcaactcag caaaagttcg atttattcaa caaagccgcc 7560
   gtcccgtcaa gtcagcgtaa tgctctgcca gtgttacaac caattaacca attctgatta 7620
   gaaaaactca tcgagcatca aatgaaactg caatttattc atatcaggat tatcaatacc 7680
   atatttttga aaaagccgtt tctgtaatga aggagaaaac tcaccgaggc agttccatag 7740
   gatggcaaga tcctggtatc ggtctgcgat tccgactcgt ccaacatcaa tacaacctat 7800
   taatttcccc tcgtcaaaaa taaggttatc aagtgagaaa tcaccatgag tgacgactga 7860
   atccggtgag aatggcaaaa gctctgcatt aatgaatcgg ccaacgcgcg gggagaggcg 7920
   gtttgcgtat tgggcgctct tccgcttcct cgctcactga ctcgctgcgc tcggtcgttc 7980
   ggctgcggcg agcggtatca gctcactcaa aggcggtaat acggttatcc acagaatcag 8040
   gggataacgc aggaaagaac atgtgagcaa aaggccagca aaaggccagg aaccgtaaaa 8100
   aggccgcgtt gctggcgttt ttccataggc tccgcccccc tgacgagcat cacaaaaatc 8160
   gacgctcaag tcagaggtgg cgaaacccga caggactata aagataccag gcgtttcccc 8220
   ctggaagctc cctcgtgcgc tctcctgttc cgaccctgcc gcttaccgga tacctgtccg 8280
   cctttctccc ttcgggaagc gtggcgcttt ctcatagctc acgctgtagg tatctcagtt 8340
   cggtgtaggt cgttcgctcc aagctgggct gtgtgcacga accccccgtt cagcccgacc 8400
   gctgcgcctt atccggtaac tatcgtcttg agtccaaccc ggtaagacac gacttatcgc 8460
   cactggcagc agccactggt aacaggatta gcagagcgag gtatgtaggc ggtgctacag 8520
   agttcttgaa gtggtggcct aactacggct acactagaag aacagtattt ggtatctgcg 8580
   ctctgctgaa gccagttacc ttcggaaaaa gagttggtag ctcttgatcc ggcaaacaaa 8640
   ccaccgctgg tagcggtggt ttttttgttt gcaagcagca gattacgcgc agaaaaaaag 8700
   gatctcaaga agatcctttg atcttttcta cggggtctga cgctcagtgg aacgaaaact 8760
   cacgttaagg gattttggtc atgagattat caaaaaggat cttcacctag atccttttga 8820
   tccggaatta attcctgtgg ttggcatgca catacaaatg gacgaacgga taaacctttt 8880
   cacgcccttt taaatatccg attattctaa taaacgctct tttctcttag gtttacccgc 8940
   caatatatcc tgtcaaacac tgatagttta aactgaaggc gggaaacgac aatctgatca 9000
   tgagcggaga attaagggag tcacgttatg acccccgccg atgacgcggg acaagccgtt 9060
   ttacgtttgg aactgacaga accgcaacgc tgcaggaatt ggccgcagcg gccatttaaa 9120
   tggtacctta attaacgtac gaagcttgca tgcacgcggt ctagagcggc cgcctcgagg 9180
   taccgggccc cccctcgagg tcgacggtat cgataagctt gcatgcctgc agtgcagcgt 9240
   gacccggtcg tgcccctctc tagagataat gagcattgca tgtctaagtt ataaaaaatt 9300
   accacatatt ttttttgtca cacttgtttg aagtgcagtt tatctatctt tatacatata 9360
   tttaaacttt actctacgaa taatataatc tatagtacta caataatatc agtgttttag 9420
   agaatcatat aaatgaacag ttagacatgg tctaaaggac aattgagtat tttgacaaca 9480
   ggactctaca gttttatctt tttagtgtgc atgtgttctc cttttttttt gcaaatagct 9540
   tcacctatat aatacttcat ccattttatt agtacatcca tttagggttt agggttaatg 9600
   gtttttatag actaattttt ttagtacatc tattttattc tattttagcc tctaaattaa 9660
   gaaaactaaa actctatttt agttttttta tttaataatt tagatataaa atagaataaa 9720
   ataaagtgac taaaaattaa acaaataccc tttaagaaat taaaaaaact aaggaaacat 9780
   ttttcttgtt tcgagtagat aatgccagcc tgttaaacgc cgtcgacgag tctaacggac 9840
   accaaccagc gaaccagcag cgtcgcgtcg ggccaagcga agcagacggc acggcatctc 9900
   tgtcgctgcc tctggacccc tctcgagagt tccgctccac cgttggactt gctccgctgt 9960
   cggcatccag aaattgcgtg gcggagcggc agacgtgagc cggcacggca ggcggcctcc 10020
   tcctcctctc acggcacggc agctacgggg gattcctttc ccaccgctcc ttcgctttcc 10080
   cttcctcgcc cgccgtaata aatagacacc ccctccacac cctctttccc caacctcgtg 10140
   ttgttcggag cgcacacaca cacaaccaga tctcccccaa atccacccgt cggcacctcc 10200
   gcttcaaggt acgccgctcg tcctcccccc ccccccctct ctaccttctc tagatcggcg 10260
   ttccggtcca tggttagggc ccggtagttc tacttctgtt catgtttgtg ttagatccgt 10320
   gtttgtgtta gatccgtgct gctagcgttc gtacacggat gcgacctgta cgtcagacac 10380
   gttctgattg ctaacttgcc agtgtttctc tttggggaat cctgggatgg ctctagccgt 10440
   tccgcagacg ggatcgattt catgattttt tttgtttcgt tgcatagggt ttggtttgcc 10500
   cttttccttt atttcaatat atgccgtgca cttgtttgtc gggtcatctt ttcatgcttt 10560
   tttttgtctt ggttgtgatg atgtggtctg gttgggcggt cgttctagat cggagtagaa 10620
   ttctgtttca aactacctgg tggatttatt aattttggat ctgtatgtgt gtgccataca 10680
   tattcatagt tacgaattga agatgatgga tggaaatatc gatctaggat aggtatacat 10740
   gttgatgcgg gttttactga tgcatataca gagatgcttt ttgttcgctt ggttgtgatg 10800
   atgtggtgtg gttgggcggt cgttcattcg ttctagatcg gagtagaata ctgtttcaaa 10860
   ctacctggtg tatttattaa ttttggaact gtatgtgtgt gtcatacatc ttcatagtta 10920
   cgagtttaag atggatggaa atatcgatct aggataggta tacatgttga tgtgggtttt 10980
   actgatgcat atacatgatg gcatatgcag catctattca tatgctctaa ccttgagtac 11040
   ctatctatta taataaacaa gtatgtttta taattatttt gatcttgata tacttggatg 11100
   atggcatatg cagcagctat atgtggattt ttttagccct gccttcatac gctatttatt 11160
   tgcttggtac tgtttctttt gtcgatgctc accctgttgt ttggtgttac ttctgcaggt 11220
   cgactctaga ggatccagaa ttcgtgatca aatggccgca acaagcagca caagcagcca 11280
   gtcttttgac atagagctcg acatcatcgg ccagcaaccg cctcttcttt caatctacac 11340
   ccagatcagt ctcgtttacc ccgtctctga tccctcccag tatcccacca tcgtcagcac 11400
   ccttgaggaa ggcctaaaac gcctctctca aaccttccca tgggtcgcgg gccaggtcaa 11460
   gaccgagggc atcagcgaag gaaacacagg aacttccaag atcattccat atgaggagac 11520
   accccgtctt gtggtgaaag acctccgtga tgattcctca gcgccaacga tcgaggggtt 11580
   gagaaaggcg ggtttcccct tagagatgtt tgacgagaac gtcgtcgctc cgaggaagac 11640
   attagctatc ggacctggca atggccccaa cgacccgaag cctgtgttgc tattgcagct 11700
   caacttcatt aagggcggac tcattctcac cgtcaacgga caacatggtg ctatggacat 11760
   gacaggacaa gatgcaatta ttcgtcttct ctccaaggcg tgccgcaacg aatcattcac 11820
   cgaggaggaa atctcggcca tgaacctcga tcgcaagacg gtagtccctc tccttgaaaa 11880
   ctacaaagtt ggtcctgagc tagaccacca gatcgccaaa cctgcgcctg ctggcgacgc 11940
   tccacccgca ccggccaagg caagctgggc gttcttttca ttcactccca aggccctctc 12000
   ggagctgaaa gacgcagcca caaagactct tgacgcgtcg tccaagtttg tgtcaactga 12060
   tgatgctctt tcggcgttta tctggcaatc aacctcgcgc gtacgtctcg caagattgga 12120
   tgcttccaca cctactgaat tctgccgcgc tgtcgacatg cggggcccaa tgggcgtatc 12180
   aagcacatac ccaggccttc ttcaaaacat gacctaccat gactcgaccg tcgccgaaat 12240
   cgccaacgaa ccacttggcg caacagcatc acgcctgcgc tcggaactca acagtgatcg 12300
   tttgcgcaga cgaacacaag ctttggcgac gtacatgcat ggcctgcctg acaagtcgag 12360
   cgtctccctg accgccgatg cgaatccgtc aagcagcatc atgctgagtt cctgggccaa 12420
   ggtgggatgc tgggagtatg actttgggtt tggactgggt aagcctgaga gtgtgagaag 12480
   acctcgcttt gaaccttttg agagtttgat gtactttatg cccaagaagc ctgatgggga 12540
   gtttacggcg tccatttctc tgagggatga ggatatggag agactaaagg cggatgagga 12600
   gtggacaaag tacgcaaagt atattgggta gatagtttac tagactactg caggatatcg 12660
   tggatccccg aatttccccg atcgttcaaa catttggcaa taaagtttct taagattgaa 12720
   tcctgttgcc ggtcttgcga tgattatcat ataatttctg ttgaattacg ttaagcatgt 12780
   aataattaac atgtaatgca tgacgttatt tatgagatgg gtttttatga ttagagtccc 12840
   gcaattatac atttaatacg cgatagaaaa caaaatatag cgcgcaaact aggataaatt 12900
   atcgcgcgcg gtgtcatcta tgttactaga tcgggaattc ggcgcgcca           12949